# EUROPEAN PATENT SPECIFICATION

(11) **EP 4 032 458 B1**
(45) Date of publication and mention of the grant of the patent: **02.07.2025**
(21) Application number: 21214195.6
(22) Date of filing: 13.12.2021
(51) Int. Cl.: A61B 1/00, A61B 1/05, A61B 1/002, A61B 1/07, A61B 1/06, A61B 1/227, A61B 1/12

(54) **MEDICAL INSTRUMENTS**
MEDIZINISCHE INSTRUMENTE
INSTRUMENTS MÉDICAUX

(30) Priority: 25.01.2021 EP 21153353
(43) Date of publication of application: 27.07.2022
(73) Proprietor: Tympany Medical Limited, H91 YXN0 Galway (IE)
(72) Inventor: O'CALLAGHAN, Rory, Cavan (IE); MCGLOUGHLIN, Elizabeth, Co Limerick (IE); WALSH, Christina, Galway (IE); CLANCY, Neil, Co Kildare (IE)
(74) Representative: Keltie Ltd

(56) References cited:
- US-A- 6 141 037
- US-A1- 2004 143 162
- US-A1- 2007 055 103
- US-A1- 2007 282 165
- US-A1- 2016 242 635

## Description

This invention relates to medical instruments, exemplified in this specification by medical scopes such as endoscopes or otoscopes for use in examining and treating the ears.

Instruments such as endoscopes and otoscopes may be employed to examine and perform surgery on ears. Some such instruments may be held against the surgeon's eye to view and magnify the subject whereas other such instruments have a camera that allows the subject to be viewed on a video display such as a computer monitor.

Effective access and visualisation are key to safe surgical procedures. In this respect, traditional ear surgery employing microscopic visualisation suffers from the disadvantage of a narrow field of view looking down into the ear canal. As a result, there can be poor visualisation of disease in the middle ear in regions that are difficult to access, such as the sinus tympani and facial recess. Whilst trans-canal surgery may be performed through a speculum by using a microscope and specialised surgical instruments, this again suffers from a narrow field of view.

Thus, to improve the field of view, it is often necessary to expose the middle ear and attic area by performing a mastoidectomy. However, as the ears are surrounded by dense bone, mastoidectomy procedures are associated with increased operating times, potentially significant complications, longer hospital stays and protracted recovery.

Another disadvantage of microscopic visualisation in ear surgery is the substantial size of a surgical microscope. This compromises the surgeon's dexterity, restricts the space available for surgical intervention and may have negative ergonomic consequences for the surgeon when using the microscope.

Endoscopic ear surgery has become a growing clinical speciality, not least because of the drawbacks of microscopic visualisation. In comparison to a surgical microscope, an endoscope offers improved visibility and allows less invasive surgery. However, the ear presents particular challenges to the use of an endoscope, with a requirement for the image-sensing part of the endoscope to be as small as possible while maintaining image quality and allowing multiple angles of view. Another challenge relates to cleaning, especially to keeping a lens of the endoscope clean in use, and more generally to cleaning or reconditioning the endoscope for reuse.

In use, tissue or other contaminants adhering to the lens of an endoscope can obscure vision. Consequently, the endoscope may have to be removed from a subject's ear canal repeatedly during surgery for cleaning. This interrupts and prolongs the surgical procedure and increases the risk of errors or inadvertent injury to the patient.

US 2004/143162 describes an endoscope with a shaft and an interchangeable head. The head is detachably connected to the distal end of the shaft at a coupling point. A change in image quality is apparent if the head becomes loosened.

US 2007/282165 describes an endoscope providing electrical isolation between the endoscope and a camera control unit coupled to the endoscope. The endoscope uses an optical channel to transmit illuminating light to the endoscope and to transmit image data generated by an imaging device to the camera control unit.

US 6141037 describes a video camera system in which the camera head is powered at least in part by electrical energy converted from optical energy provided by a light source. In one implementation, communication between the camera head and control circuitry occurs by means of a wireless communications interface.

US 2016/242635 describes an in-vivo monitoring camera system that includes a camera support tube, a camera unit, a control system and a display apparatus.

US 2007/055103 describes an endoscope comprising an elongated rigid shaft and an optical imaging system arranged at a distal end of the shaft for receiving light from an observation area. The optical imaging system is pivotable with respect to the shaft about a first pivot axis transverse to a longitudinal axis of the shaft. The optical imaging system is also pivotable with respect to the shaft about a second pivot axis that is spaced from the first pivot axis and is also transverse to the longitudinal axis of the shaft.

In relation to reuse, there is a risk that an endoscope could be contaminated with prions that may be present in the ear canal. Contamination with the wrong type of prions could endanger a patient's life. Even cleaning and sterilisation processes used in industry cannot remove certain types of contamination such as prions; in this respect, prions cannot be killed as they are not living organisms. Currently, therefore, the World Health Organisation recommends that all instruments used in surgery are discarded after surgery and so are single use wherever possible.

In view of this and other risks, single-use endoscopes for ear surgery have appeared on the market. Indeed, there is now a significant trend for hospitals and manufacturers to move from a model of reuse and maintenance of endoscopic equipment to single-use endoscopy. This trend has been driven to some extent by the inability of in-hospital sterilisation to sterilise equipment fully, hence reducing confidence in the system. Unlike simple single-use hospital equipment, such as scalpel blades and syringes, single-use endoscopes are large and generate significant amounts of medical waste. Medical waste is typically incinerated, rendering materials irrecoverable. In the case of instruments containing electronics, this can include precious metals or rare earth metals. The result is a waste of valuable resources and potentially a negative impact on the environment.

At first sight, moving from a single-use system to a circular economy system could be seen as a solution to this problem. For a manufacturer of single-use instruments, moving to a circular economy system would firstly involve recovering used instruments from a hospital in a reverse logistics operation. This recovery step would be followed by: dismantling the instruments; identifying components that can be reused and cleaning them; discarding components that cannot be reused and replacing them with new components; assembling a new instrument from the reused and new components; and packing and sterilising the new instrument ready for distribution. However, this process is not feasible as an alternative to single-use endoscopy without incorporating special design features in the endoscope and in the processes used in the circular economy system.

The invention addresses how a surgical instrument may be designed for integration into a circular economy system in which at least a substantial portion of the instrument can be reused safely and cost-effectively, with minimal waste. The inventive concept also embraces a method by which a surgical instrument can be reconditioned for reuse under a circular economy system.

More generally, the invention also improves prior solutions to provide a panoramic endoscope for ear surgery that is compact, reliable and easy to keep clean in use and that provides highly-effective visualisation.

Embodiments of the invention describe how a camera of an endoscope can be integrated into a rotating head to satisfy the requirement of enabling multiple angles of view. Ideally, the rotating head can turn through 360°. This makes it convenient to clean the lens of the camera by rotating the head and hence the lens past a useful viewing angle and over a wiper blade that removes debris from the lens. Elegantly, this allows one simple mechanism to solve two of the major challenges of endoscopic ear surgery.

To allow an endoscope to operate in this way, the camera unit of the rotating head is an integrated module that incorporates a lens, an image sensor and data transmission provisions. Data may be transmitted wirelessly from the rotating head to a supporting probe structure of the endoscope or to a control unit outside the endoscope. A wireless solution has the advantage of negating wired data connections that could otherwise limit rotation of the head.

Electrical power must also be delivered to the rotating head to power the camera module and any other electrical systems such as illumination LEDs, which commonly are found alongside chip-on-tip image sensors in endoscope designs. Again, this should ideally be done in a way that does not limit rotation of the head.

One solution for delivering electrical power to a rotating head involves the use of rotating or sliding contacts on or around the axis of rotation, where the head is joined to the supporting probe of the endoscope. This requires a robust sealing arrangement to insulate the live contacts from moisture in the ear and from saline solution used in surgery, which is electrically conductive. Another solution is to deliver power wirelessly or electromagnetically, for example in a manner akin to an inductive charging unit. However, implementing either of these solutions remains challenging due to the compactness that is required of an endoscope for ear surgery.

The narrowness of the ear canal determines that, optimally, the outer diameter of the probe and the rotating head should be no greater than 3.2mm. Wireless power delivery requires a coil in the rotating head that, in this context, could significantly restrict the space available for other components in the head, notably the camera module itself. The same size constraints also have a significant impact on image quality as they dictate that the image sensor of the camera must be small in area, typically smaller than 2mm x 2mm. This in turn means a small pixel size; and with higher resolution, the pixel size gets smaller still. A problem then ensues because smaller sensor pixels are less sensitive to light than larger pixels. This results in a darker and lower-quality image than would be delivered by a larger sensor, all other things being equal. To counteract this, a larger optical aperture or lens can be used to capture more light but this has the knock-on effect of a reduced depth of field. Indeed, in sufficiently compact optical systems, the depth of field can become so restricted as to be unusable for surgery. Ultimately, what is needed to offset the small size of the system is better illumination. This can require the provision of bigger and more powerful LEDs or the use of a remote light source and optical fibres or other light transmission features.

Against this background, the invention resides in a medical scope as defined in claim 1 that comprises: an imaging head supported by and movable relative to a body about an axis of rotation extending from the body, the imaging head including an image sensor and a light emitter for illuminating a field of view of the image sensor; a light path extending from the body parallel to the axis of rotation to the light emitter, the light path including a light inlet of the imaging head opposed to a light outlet of the body across a wireless interface between the imaging head and the body; and a light transmitter in the imaging head, the light transmitter being coupled to the light path to transmit image data from the image sensor to the body across the interface.

Elegantly, the light inlet and the light outlet may be mutually opposed across a mount at which the imaging head is movably attached to the body. For example, the light inlet and the light outlet may be disposed on said axis of rotation about which the imaging head can turn relative to the body. In a compact arrangement, the light emitter may be disposed between the interface and the image sensor in a direction parallel to the axis of rotation, at a position spaced radially from the axis of rotation.

The interface may further comprise a wireless power transmission coupling effecting transmission of power from the body to the image sensor. The power transmission coupling may, for example, comprise mutually-opposed coils, a first of those coils surrounding the light inlet of the imaging head and a second of those coils surrounding the light outlet of the body. In an alternative approach, the imaging head may include a photovoltaic generator for powering the image sensor from light transmitted across the interface.

The imaging head may further comprise a filter in the light path, configured to block reflected light from the light emitter and to pass light transmitted from the light transmitter.

A light source outside the imaging head may be coupled to the light path to provide illuminating light to the light emitter. In that case, the light source and the image sensor may be synchronised such that the light source is on when the image sensor is capturing an image frame and the light source is off between image frames while the light transmitter transmits imaging data from the image sensor.

An image sensor controller outside the imaging head may be arranged to convey a control signal from the image sensor controller across the interface to the image sensor. The control signal may comprise a clock signal multiplexed with a write signal.

The image sensor controller may, for example, be connected to the wireless power transmission coupling of the interface to convey the control signal to the image sensor through that coupling. The image sensor controller could instead drive an LED to convey the control signal to the image sensor as an optical control signal. In that case, the light transmitter in the imaging head may be responsive to the optical control signal.

The present invention also concerns a method of operating a scope that comprises an imaging head movable relative to a supporting body about an axis of rotation extending from the body as defined in claim 15. The method comprises: illuminating a field of view of the imaging head by conveying light along a light path parallel to said axis of rotation across a wireless interface from the body to the imaging head; and transmitting image data optically along the light path across the interface from the imaging head to the body.

In some examples, the medical scope of present disclosure comprises: an elongate body; an imaging head supported by and movable relative to a mount of the body, the imaging head including a light emitter for illuminating a field of view of the imaging head; and a light path extending from the body and through the imaging head to the light emitter, the light path including a light inlet of the imaging head opposed to a light outlet of the body. The imaging head is preferably cantilevered from the mount. Conveniently, the light inlet and the light outlet may be mutually opposed across a pivot coupling at which the imaging head is movably attached to the mount.

Where the imaging head contains an image sensor, the imaging head may further comprise a photovoltaic generator arranged such that impingement of light generates electrical power for the image sensor. The imaging head may further comprise a data transmitter powered by the photovoltaic generator to transmit image data from the image sensor to a receiver outside the imaging head.

The imaging head may also comprise a light guide in the light path, configured to direct a portion of light received from the body onto the photovoltaic generator and another portion of light received from the body to the light emitter. The light guide may, for example, comprise a filter that is configured to divide the light received from the body into said portions to be directed onto the photovoltaic generator and toward the light emitter.

Where the light inlet and the light outlet are disposed on an axis of rotation about which the imaging head can turn relative to the mount, the light guide may be disposed between the mount and the image sensor in a direction parallel to that axis.

The imaging head may comprise encapsulation around the image sensor. That encapsulation may include a disassembly interface at which the image sensor is preferentially separable from the light emitter. The disassembly interface may, for example, comprise at least one point of weakness in the encapsulation. The disassembly interface may also, or instead, comprise a protective strip embedded along a cutting line in the encapsulation.

The present disclosure also provides an exemplary method of illuminating a field of view of an imaging head of a medical scope, which head is movable relative to a supporting body of the scope. The method comprises conveying light along a light path from a light outlet of the body to a light inlet of the imaging head and then through the imaging head to be emitted from the imaging head. Elegantly, as noted above, an image sensor of the imaging head may be powered with electricity generated within the imaging head from at least a portion of the light conveyed along the light path.

Light travelling along the light path within the imaging head may be filtered to separate that light into components for power generation and for illumination, respectively. At least one of those components of the light may be varied, for example in intensity, relative to another of those components of the light to enable independent adjustment of power generation and/or illumination.

Examples of the present disclosure deliver light longitudinally through a supporting structure to a rotating endoscope head. Light is used as a mechanism for power delivery to a chip-on-tip endoscope for ear surgery.

In some examples, sealed capsules are used to protect components designed for disassembly in a circular economy system. Provision may be made for daisy-chaining for transmission of light, power or data between the capsules.

In order that the invention may be more readily understood, reference will now be made, by way of example, to the accompanying drawings in which:
Figure 1 is a perspective view of a distal end portion of an endoscope of the invention;
Figure 2 is a side view of the endoscope of Figure 1 in longitudinal section;
Figure 3 corresponds to Figure 2 but shows the endoscope from a direction orthogonal to that shown in Figure 2 about a longitudinal axis;
Figure 4 shows a variant of the endoscope but otherwise corresponds to Figure 2;
Figure 5 is a block diagram of an endoscope system comprising the variant of Figure 4;
Figure 6 corresponds to Figure 2 and shows how an endoscope of the invention may be designed to facilitate remanufacture in a circular economy system;
Figure 7 is a sequence of schematic drawings that illustrate how the invention may be applied in a circular economy system;
Figure 8 is a perspective view of a tray for use in the method illustrated in Figure 7;
Figure 9 is an enlarged detail view of a robotic cell used in one of the steps of the method illustrated in Figure 7;
Figures 10a to 10d are side views in longitudinal section of camera module variants for use in endoscopes of the invention;
Figure 11 is a schematic side view of a drive mechanism for rotating the head of an endoscope of the invention;
Figures 12a to 12g are simplified versions of Figure 11, showing how the drive mechanism operates;
Figures 13 to 17 are side views of further examples, all in longitudinal section;
Figure 18 is a side view in longitudinal section of a distal portion of a probe showing an optical pathway and components for transmission of data and power;
Figure 19 is a block diagram of an endoscope system comprising the variant of Figure 18;
Figure 20 is a side view in longitudinal section of a distal portion of a probe in which data is transferred optically and power is transferred wirelessly but non-optically;
Figures 21a and 21b are detail perspective views of the probe of Figure 20 in assembled and disassembled forms;
Figure 22 is an exploded perspective view of a rotating head assembly for the probe of Figures 20, 21a and 21b;
Figure 23 is an enlarged perspective view of a camera module of the rotating head assembly shown in Figure 22;
Figure 24 is a block diagram of an endoscope system comprising the variant of Figure 20;
Figure 25 is a block diagram of an endoscope system that addresses two-way data transmission;
Figure 26 is a diagram that illustrates data multiplexing;
Figures 27a and 27b are perspective views of an endoscope system of the invention, comprising a probe assembly, a handle assembly and a camera control unit;
Figure 28 is a sectioned perspective view of a proximal end of the probe assembly shown in Figures 27a and 27b; and
Figures 29(a) to 29(f) are a sequence of perspective views showing some of the steps involved in manufacturing the endoscope.

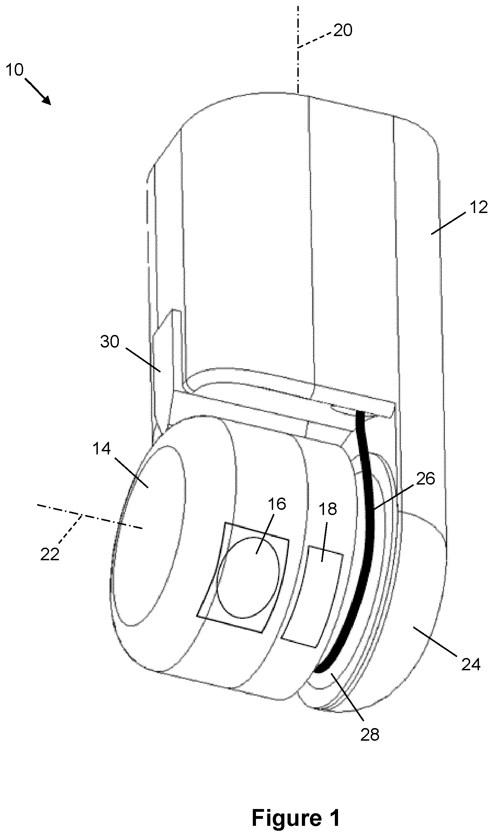

Referring firstly to Figures 1 to 3 of the drawings, a distal end portion of an endoscope 10 comprises a body in the form of an elongate stem, shaft or probe 12 that supports a rotating imaging head 14. The head 14 houses an integrated camera module 16 and a light emitter 18 disposed beside the camera module 16 to illuminate the field of view.

The probe 12 extends along a longitudinal axis 20. The head 14 turns relative to the probe 12 about an axis of rotation 22 that extends orthogonally with respect to the longitudinal axis 20. The camera module 16 has a field of view that extends radially with respect to the axis of rotation 22. Thus, the camera module 16 can be oriented through multiple angles of view by turning the probe 12 around its longitudinal axis 20 and by turning the head 14 around its axis of rotation 22. In Figure 1, the camera module 16 and the adjacent light emitter 18 are shown at an angle of 45° to the longitudinal axis 20.

The head 14 is generally cylindrical, extending along and being rotationally symmetrical about the axis of rotation 22. The head 14 is supported for rotation by a mount in the form of a distally-extending cantilever arm 24 of the probe 12, offset laterally from the longitudinal axis 20. The cantilever arm 24 supports the head 14 at only one end or side of the head 14. This beneficially maximises the size of the head 14 within the constrained diameter of the endoscope 10, eases assembly and disassembly, provides greater freedom to arrange the internal components of the head 14 and leaves the free end or side of the head 14 available for additional lateral imaging if desired.

In this example, rotation of the head 14 is driven by a continuous drive cable 26 that extends from a drive mechanism (not shown) within the probe 12 and wraps around the head 14. The drive cable 26 is retained in a pulley groove 28 encircling the axis of rotation 22 adjacent to the end of the head 14 adjoining the cantilever arm 24. Rotation of the head 14 relative to the probe 12 about the axis of rotation 22 is unconstrained and so can exceed 360° in this example.

The probe 12 supports a distally-facing resilient wiper blade 30 that has an elongate free edge extending parallel to the axis of rotation 22. The wiper blade 30 bears against the proximal side of the head 14. As the head 14 is rotated past the wiper blade 30, the wiper blade 30 cleans debris from the head 14 to prevent that debris obscuring the field of view of the camera module 16 or blocking illumination from the light emitter 18.

Although not shown here, an outer lens of the camera module 16 may sit slightly proud of the surrounding outer surface of the head 14 so that the wiper blade 30 only impinges on the outer lens as the head 14 turns and does not drag on the remaining circumference of the head 14. Thus, there may be a small radial clearance between the wiper blade 30 and the head 14 except where the protruding outer lens of the camera module 16 bridges that gap on encountering the wiper blade 30.

In this embodiment, as will now be explained, the light emitter 18 is the distal end of an illumination conductor or light path that conveys light along the probe 12 to the head 14 from a remote light source. This solution is favoured in view of size constraints within the head 14 and because it allows easier variation of the light spectrum, for example by use of different light sources, which may be useful for tissue identification and in other respects.

Unlike transmission of electricity, transmission of light from the probe 12 into the head 14 does not require a complex contact or sealing arrangement. However, the problem of providing electrical power to the camera module 16 within the head 14 remains. In this embodiment, the solution is to generate the necessary electricity within the head 14 from a portion of the incoming light. In this way, a single efficient mechanism avoids the problem of sealing electrical contacts and better addresses the challenge of providing an illumination solution within a restricted space than the alternative of integral LEDs in the head. The mechanism shown also facilitates the abovementioned rotation and cleaning solutions.

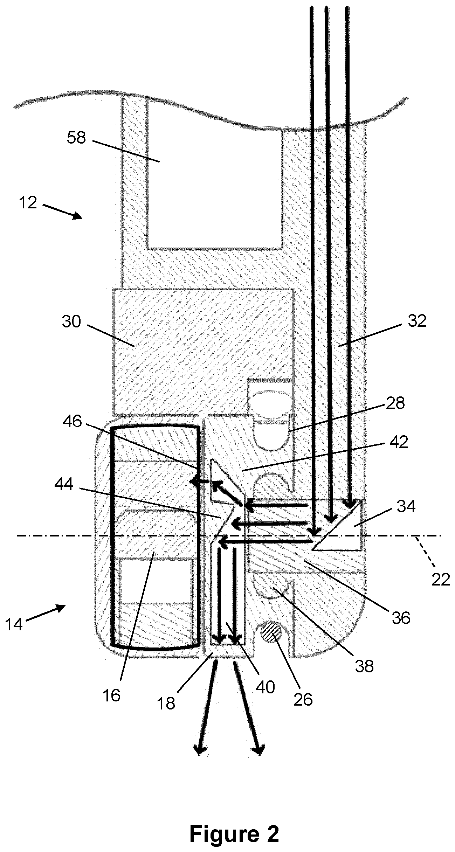

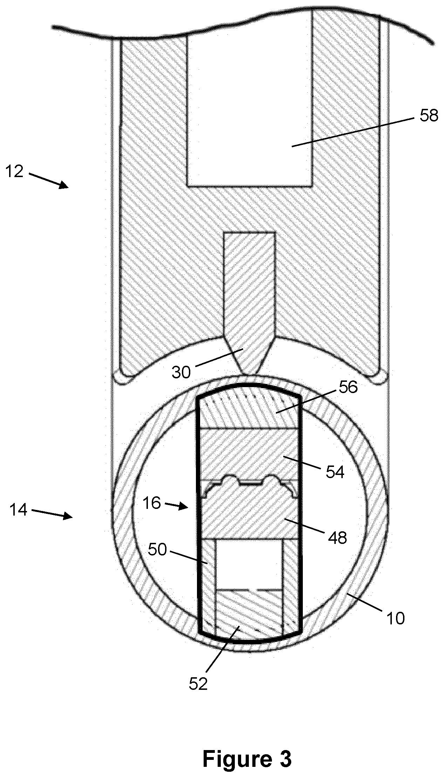

The cross-sectional views of Figures 2 and 3 show internal features that define light transmission paths within the endoscope 10.

Figure 2 is a view from a direction orthogonal to both the longitudinal axis 20 and the axis of rotation 22. This shows that light from an external light source (not shown) travels distally through an optical medium 32 in the probe 12, in a direction generally parallel to the longitudinal axis 20. On reaching the distal end portion of the probe 12, the light encounters a mirror or preferably a prism 34 mounted in the cantilever arm 24. This directs the light through an optical medium 36 in a direction generally parallel to the axis of rotation 22 to enter the head 14 via the sliding interface or gap at the junction between the cantilever arm 24 and the head 14. The optical medium 36 therefore serves as a light outlet from which light is conveyed or transmitted across the interface or gap to impinge on the head 14.

Figure 2 also shows that the cantilever arm 24 comprises a spigot 38 that is engaged within a complementary socket of the head 14 to support the head 14 for rotation relative to the cantilever arm 24. The optical medium 36 extends within the spigot 38 along the axis of rotation 22 to the interface between the cantilever arm 24 and the head 14, thereby to emit light into the head 14 across that interface. In this example, the spigot 38 is substantially coplanar with the groove 28 in the exterior of the head 14 that accommodates the drive cable 26.

It will be apparent from Figure 2 that the head 14 further comprises a light guide 40 behind the light emitter 18. Conveniently, the pulley component 42 defining the pulley groove 28 that receives the drive cable 26 and the socket that engages the spigot 38 of the cantilever arm 24 is integral with the light guide 40, for example as part of the same component or with internal features encapsulated within external structures.

The light guide 40 is disposed between the camera module 16 and the cantilever arm 24. In other words, the cantilever arm 24, the light guide 40 and the camera module 16 are disposed in succession along the axis of rotation 22. Thus, the light guide 40 defines part of the interface between the head 14 and the cantilever arm 24 and receives the light from the optical medium 36 within the spigot 38 of the cantilever arm 24. The light guide 40 therefore serves as a light inlet of the head 14 that receives light conveyed or transmitted from the light outlet of the cantilever arm 24 across the interface or gap at the junction between the cantilever arm 24 and the head 14.

On entering the head 14, the incoming light is split into two portions by the light guide 40. A first portion of the light impinges on a mirror or preferably a prism 44 of the light guide 40 and is thereby redirected radially away from the axis of rotation 22. That first portion of the light, thus redirected, is then conveyed through an optical medium of the light guide 40 to the light emitter 18, where it exits the head 14 to illuminate the field of view of the camera module 16.

A second portion of the light is directed through the light guide 40 to impinge on a photovoltaic cell 46 that thereby generates electrical power for the electronic components of the camera module 16. In this example, the photovoltaic cell 46 is conveniently integrated into the camera module 16 but in principle, the photovoltaic cell 46 could be separate from the camera module 16.

Reference is now also made to Figure 3, which is a view from a direction orthogonal to the longitudinal axis 20 and along the axis of rotation 22, in longitudinal section through the camera module 16. This shows that the camera module 16 preferably has a stacked configuration. Specifically, the camera module 16 suitably comprises a commercially-available image sensor 48, onto which a lens barrel 50 and a lens 52 are mounted. The image sensor 48 has BGA (ball grid array) connections that are electrically connected to a power IC (integrated circuit) 54 to convey power to the image sensor 48 and to convey image data from the image sensor 48. The image sensor 48 could include image processing circuits, in which case fewer connections may be required to transmit image data to the power IC 54.

The power IC 54 preferably includes the photovoltaic cell 46 and power control circuity to power the image sensor 48 and wireless data transmission circuitry implemented on a data IC 56. The power IC 54 also passes through data from the image sensor 48 to the data IC 56. The data IC 56 preferably includes an antenna and circuitry to transmit data in packets as required by wireless transmission protocols.

To minimise the power required for wireless data transmission, the data IC 56 of the camera module 16 conveniently transmits data from the image sensor 48 over a very short range, in this example to a wireless receiver 58 located in the probe 12 near the distal end of the probe 12.

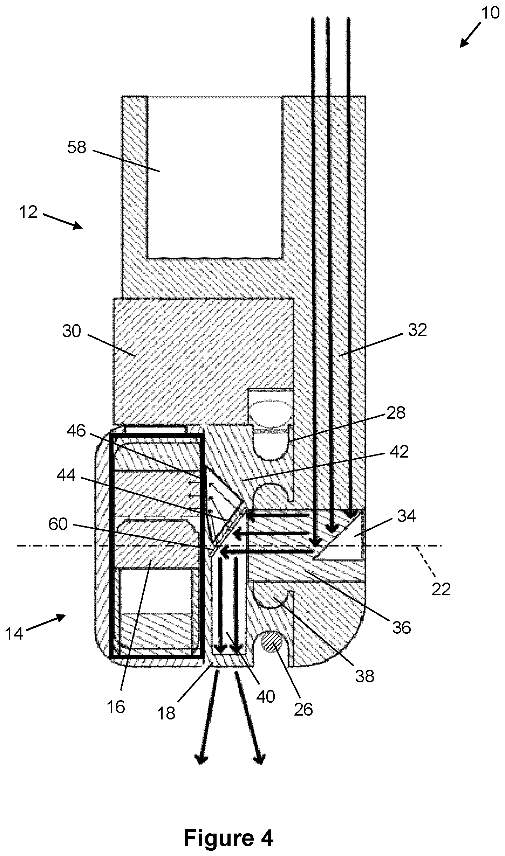

Turning now to Figure 4, this shows a variant of the embodiment shown in Figures 1 to 3 in which like numerals are used for like features. This variant differs by the addition of a filter or filter layer 60 applied to the prism 44 within the light guide 40 of the head 14. In this variant, the camera module 16 is powered by light that is tuned or filtered to a specific wavelength or spectrum, preferably outside the visible light spectrum. The filter layer 60 allows that specified light to pass through to the photovoltaic cell 46 of the camera module 16 while deflecting the remaining light to provide illumination via the light emitter 18. This allows independent control of the power transmitted to the camera module 16 and the visible light used for illumination of the target area, for example by varying the intensity or other parameter of one component of the supplied light relative to the other component of that light. More generally, filtering of the light can be done inside or outside the head 14 or the probe 12.

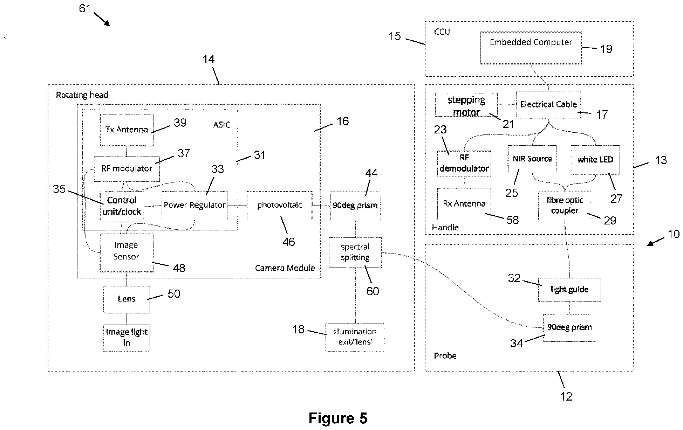

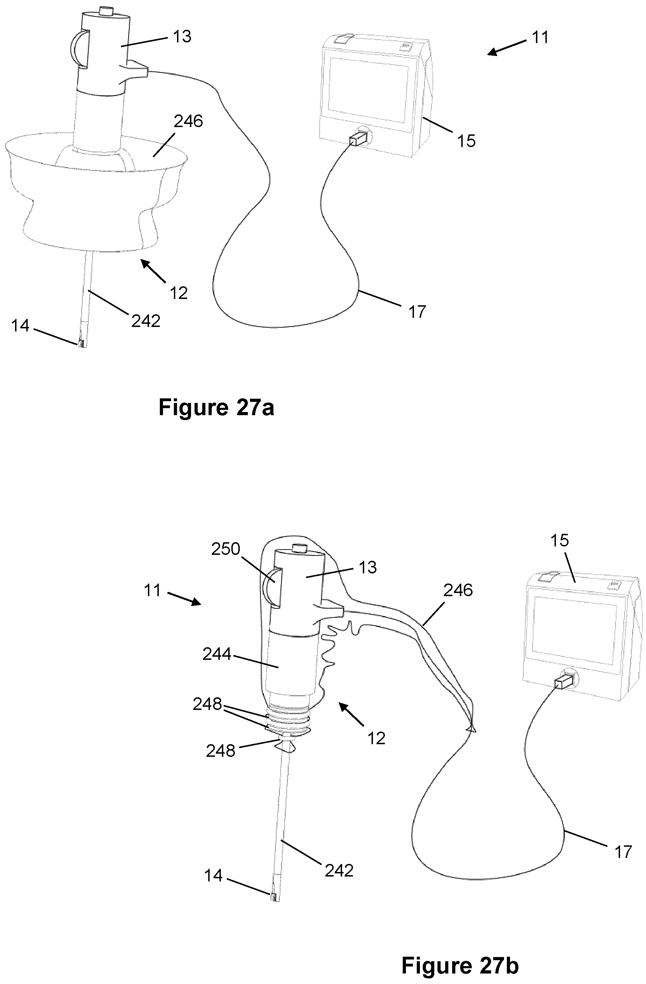

Figure 5 is a block diagram of an endoscope system 11 comprising the endoscope 10 shown in Figure 4, in which power is transferred optically from the probe 12 to the head 14 and data is transferred wirelessly from the head 14 to the probe 12 using RF. As also shown in Figures 27(a) and 27(b), the endoscope system 11 comprises a handle 13 that is removably attachable to the probe 12 and a camera control system 15 that is removably coupled to the handle 13 by an electrical cable 17 to power and control the handle 13 and the endoscope 10. For this purpose, the camera control system 15 comprises an embedded computer 19.

The handle 13 comprises a stepping motor 21 that activates a drive mechanism within the probe 12 to drive rotation of the head 14 about the axis of rotation 22 shown in Figure 4. The handle 13 further comprises a wireless receiver in the form of a receiving antenna 58 that provides signals to an RF demodulator 23. The demodulator 23, in turn, returns signals to the embedded computer 19 of the camera control system 15 via the cable 17. The cable 17 also provides power to a near-infrared source 25 and to an LED 27 that emits white light. The outputs of the source 25 and the LED 27 are combined by a fibre optic coupler 29 and fed through the probe 12 via the aforementioned optical medium serving as a light guide 34 and then to the head 14 via the prism 34. In the head 14, spectral splitting is effected by the filter layer 60 whereby white light is directed to the light emitter 18 and near-infrared wavelengths are directed via the prism 44 to the photovoltaic cell 46 of the camera module 16.

In this example, the photovoltaic cell 46 of the camera module 16 powers an application-specific integrated circuit (ASIC) 31 that combines the functions of the aforementioned power IC 54 and data IC 56. The ASIC 31 comprises a power regulator 33, a control unit 35 with clock functionality, and an RF modulator 37 that receives signals from the image sensor 48 and that feeds signals to a transmitting antenna 39.

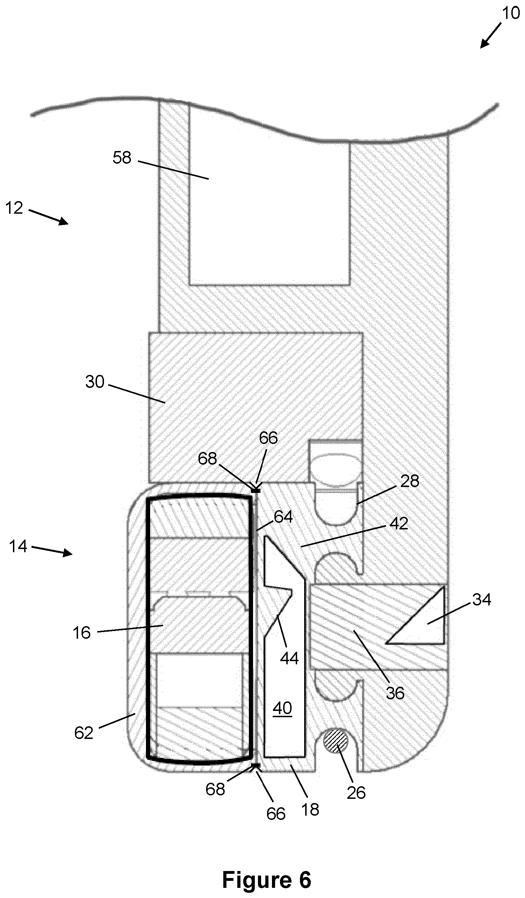

Figure 6 shows how the embodiment of Figures 1 to 3 may have features to facilitate reuse of key components in a circular economy system. Again, like numerals are used for like features. Similar principles of construction may be applied to other embodiments, such as that shown in Figure 4.

In view of the aforementioned problem of prion contamination, any structure defining an external surface or other surface of the endoscope 10 that could come into contact with organic material cannot be reused and must be removed from the device while maintaining the integrity of internal components that are not contaminated. For this purpose, the internal components are encapsulated. This ensures that once the endoscope 10 has been successfully dismantled and the encapsulation has been removed, the internal components that are to be reused cannot contaminate other components during reassembly, or in case of failure of the device and exposure of those components during surgery.

As shown in Figure 6, the major active components of the head 14 including the integrated camera module 16 and the internal features of the light guide 40, such as the prism 44, are encapsulated to form a hermetic seal around those internal components. Specifically, the camera module 16 is encapsulated by a shroud 62 and as noted above, the light guide 40 is integrated with, or solidly encapsulated into, the component that also defines the pulley groove 28 and the spigot 38.

The shroud 62 interfaces with the light guide 40 at a planar joint 64 to assemble the head 14 as a single capsule. A seal between the light guide 40 and the shroud 62 is formed by over-moulding or optionally by bonding, welding or compression. The components within the light guide 40 and the shroud 62 are likewise over-moulded or optionally glued, welded or otherwise encapsulated using a compression fit.

A groove 66 surrounding the head 14 in alignment with the joint 64 creates a line of weakness at which the capsule can be broken to separate the light guide 40 and the shroud 62 in a controlled manner to remove the protected components inside. More generally, the groove 66 exemplifies a disassembly interface at which the camera module 16 is preferentially separable from other parts of the head 14 such as the light guide 40 and/or the light emitter 18. The groove 66 is external to the head 14 in this example but optionally, a line or point of weakness could be defined within the head 14. Optionally, the groove 66 can be shaped to provide a hinge for controlled opening of the capsule, for example if the groove 66 does not extend around the entire circumference of the head 14.

Figure 6 also shows an optional strip or band 68 of a harder material embedded within the head 14 under a cutting line, for example located under the groove 66 as shown. This allows a laser or knife to cut into the head 14 while protecting the internal components of the head 14 during the cutting process. For example, a laser could cut into a plastic capsule of the head 14 to open or weaken it, while being prevented from penetrating to the inside of the capsule by a metal band 68 located under the cut site.

Advantageously, the capsule of the head 14 may be opened by applying a bending or twisting force to its outer surface to tear, crack or break the capsule. Optionally, this action may be directed to a predetermined line or point of weakness as described above and/or may follow the application of a cut or scribe mark to the capsule as a preliminary step. Ideally, the required forces are applied to the outer surface of the capsule in a controlled and repeatable manner, for example by using a robotic arm.

Confirming that the capsule of the head 14 maintained its integrity during use is important to determine whether or not a component within that capsule can be used again. During disassembly, this check can be done visually, optionally by applying strain or colour dies or scanning frequencies to visualise cracks or to check resonance. Alternatively, or additionally, liquid or gas pressure could be applied to the outer surface of the capsule. If the capsule has failed, a void or pocket in the pressurised capsule would fill with fluid. Optionally testing can be in the form of a bubble leak test conducted with a pressure differential. This can be detected by visual monitoring or by monitoring pressure.

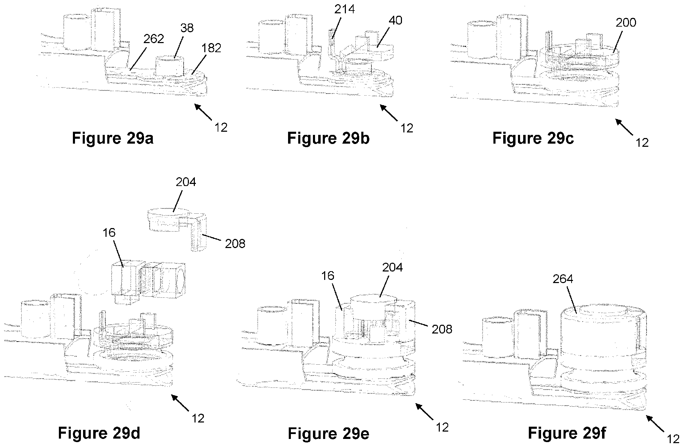

Manufacture of an endoscope 10 with encapsulated components will be described later with reference to Figures 29(a) to 29(f) of the drawings.

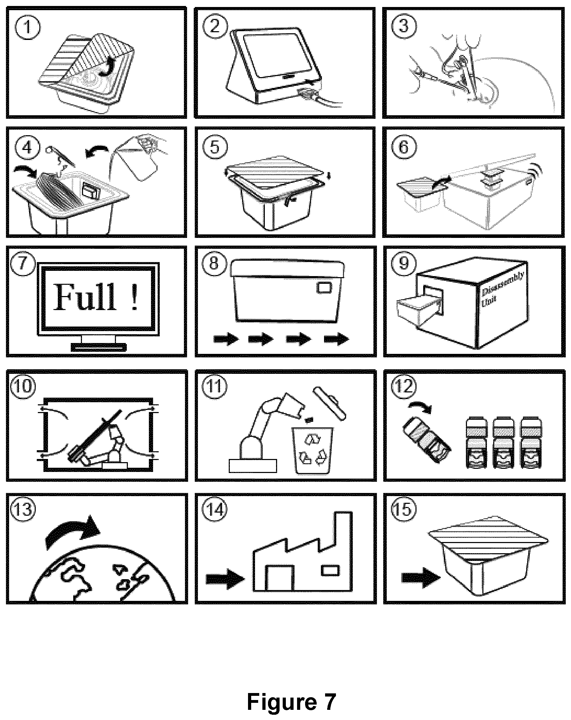

Figure 7 exemplifies how the invention may be applied in a circular economy system. In this respect, it is important to protect the integrity of key components after use and to return them. Robust reverse logistics and supply chain management and focused product design and disassembly/reassembly processes are therefore required.

Figure 7 shows an endoscope of the invention being removed from a sterile reusable tray (1), connected to a monitor or camera control unit (2) and used in ear surgery (3). After use, the endoscope is placed back in the original tray (4), although the tray in which the endoscope is supplied or returned could instead be single-use.

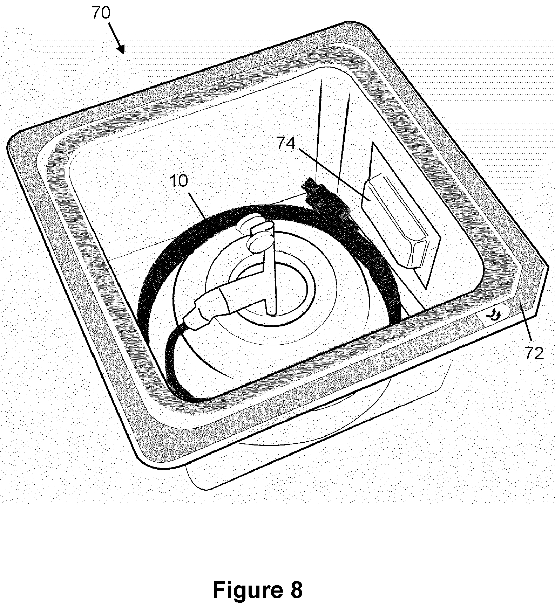

An example of a reusable tray 70 is shown in Figure 8. In step (5) of Figure 7, a user pulls a protective backing from a return seal 72 and then reseals the tray 70 around an endoscope 10, conveniently using the original covering film to close the open top of the tray 70. Advantageously, the user may firstly fill the tray 70 with water, thus activating a water-soluble detergent capsule 74 in the tray 70. The detergent initiates the cleaning process, loosening overt biological matter and debris from the endoscope 10.

Returning to Figure 7, the tray 70 is then placed in a depository provided to individual hospitals (6). Depositories may provide geolocation and inventory data. When the depository is full, or nearly so, an alert is generated (7), leading to the depository being transported (8) to a specialised disassembly unit (9). This removes the burden of cleaning and waste management from hospitals and eliminates the risk of poor sterilisation.

A global product identification system allows individual serialisation and secure tracking of products and components and validation of the authenticity of those items as part of the returns process. This enables robust implementation of a smart Kanban (lean scheduling) system in which local distributors can be used to transport the endoscope to the disassembly unit.

Advantageously, disassembly units may be robotic cells of a size that makes shipping easy. A set number of such cells could fit into a standard shipping container, or a cell could be implemented in a shipping container. Such cells may be placed regionally or nationally as a key part of a reverse logistics system.

Thus, used endoscopes delivered directly from the hospitals, classified as medical waste, may be fed into robotic cells that serve as disassembly units. Initial disassembly of endoscopes is automated with a pick-and-place robot to protect staff. Robotic arms unpack and dismantle the endoscopes (10) and sort the parts into: parts for recycling (11); parts for reuse in the circular economy system (12); waste for incineration and biodegradable materials.

The parts for reuse in the circular economy system are no longer classified as medical waste and can be shipped internationally (13) to a circular production facility at which endoscopes are remanufactured (14). This solution avoids the many regulatory challenges that would have to be overcome if parts were shipped internationally while still classified as medical waste.

Each endoscope undergoes rigorous testing to ensure that it meets standards of safety and sterility equivalent to those of the original endoscope and is packaged ready for resale (15).

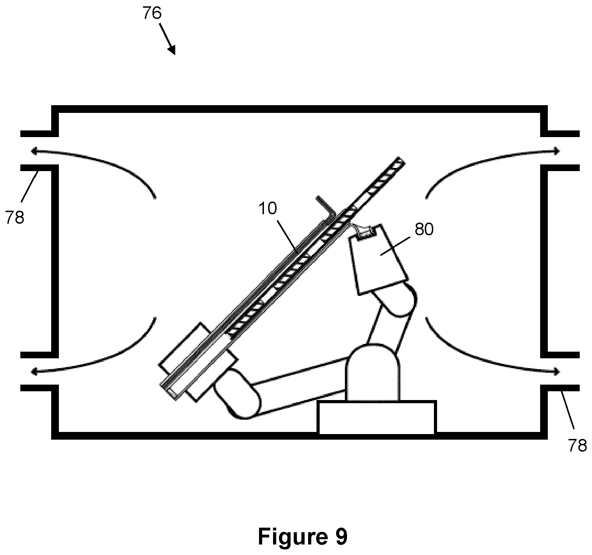

Figure 9 shows the robotic cell 76 of a disassembly unit, as may be used in step (10) of Figure 7. In this example, air is drawn from the cell 76 through ports 78 to apply a partial vacuum to the endoscope 10 during its disassembly by robotic arms 80. This is to discourage transfer of particles such as prions from the outer surface of the endoscope 10 to its internal protected components. The vacuum could be localised or transient or the entire disassembly process could take place in a low-pressure environment. Alternatively, laminar or other air flow may be applied to the endoscope 10 within the robotic cell 76 for the same purpose.

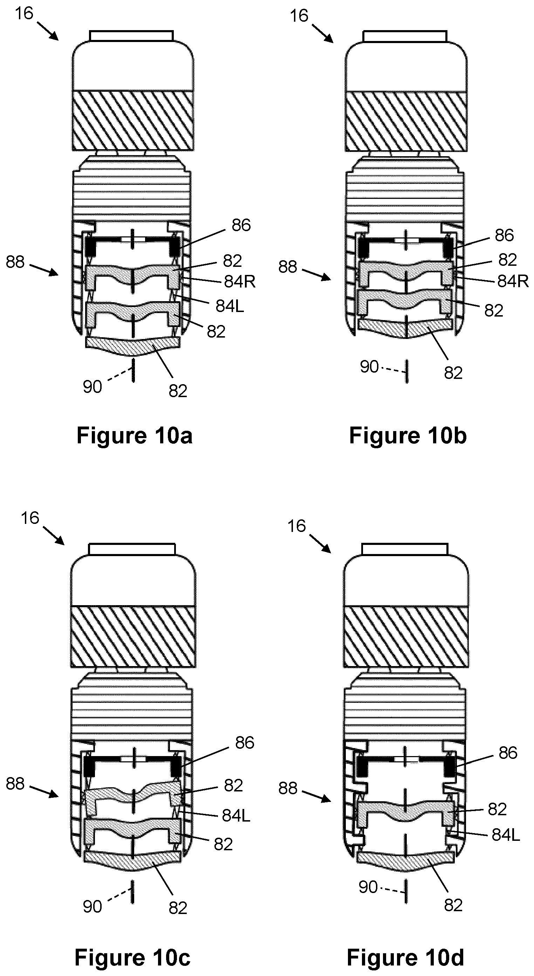

Moving on to Figures 10a to 10d, these drawings show variants of camera modules 16 that each have at least two lens elements 82, at least one of which is mounted on and movable by micro electro-mechanical system (MEMS) actuators 84. This enables the lens elements 82 to be moved independently of each other or in an accordion fashion. Optionally, the illustrated arrangements of MEMS actuators 84 could be replicated on all lens elements 82. MEMS actuators 84 could also act on a lens aperture 86 of the camera module 16 in a similar fashion.

A lens train 88 of three longitudinally-spaced lens elements 82 is shown in Figures 10a and 10b. Longitudinally-acting peripherally-positioned MEMS actuators 84L act on each lens element 82 to vary the longitudinal spacing between the lens elements 82. The effect of this is apparent from a comparison of Figure 10a with Figure 10b; the former drawing shows the lens train 88 expanded longitudinally whereas the latter drawing shows the lens train 88 contracted longitudinally. In this example, the lens elements 82 move relative to each other in accordion fashion and therefore the longitudinal positions of the lens elements 82 are inter-dependent.

Figures 10a and 10b also show that at least one lens element 82 can have further MEMS actuators 84R acting radially to keep that lens element 82, or more generally the lens train 88, in alignment. In principle, similar MEMS actuators 84R could also act on the lens aperture 86.

In Figures 10a and 10b, the lens elements 82 remain parallel and mutually aligned on a common longitudinal optical axis 90 as they move longitudinally. Conversely, the variant shown in Figure 10c illustrates how MEMS actuators 84 on opposite sides of a lens element 82 could be controlled independently, for example by extending the MEMS actuators 84L on one side and retracting the MEMS actuators 84L on the other side. This causes at least one lens element 82 to tilt relative to the optical axis 90 and to at least one other lens element 82. Similarly, extending the MEMS actuators 84R on one side and retracting the MEMS actuators 84R on the other side will cause at least one lens element 82 to move transversely relative to the optical axis 90 and to at least one other lens element 82.

These pivoting and translational movements could be used to manoeuvre or manipulate a lens train 88 containing complex asymmetric lenses, allowing refocusing in a way that is not possible with simple lenses. For example, a lateral shift of a lens element 82 could bring a different optical shape onto the optical axis, thus refocusing the optical system.

In the variant shown in Figure 10d, the lens train 88 comprises two lens elements 82. The lens elements 82 are suspended independently via MEMS actuators 84L and therefore are movable relative to a surrounding barrel structure independently of each other. The lens aperture 86 could also be mounted in a similar way. Again, provisions may be made for pivoting and/or translational movements like those described above.

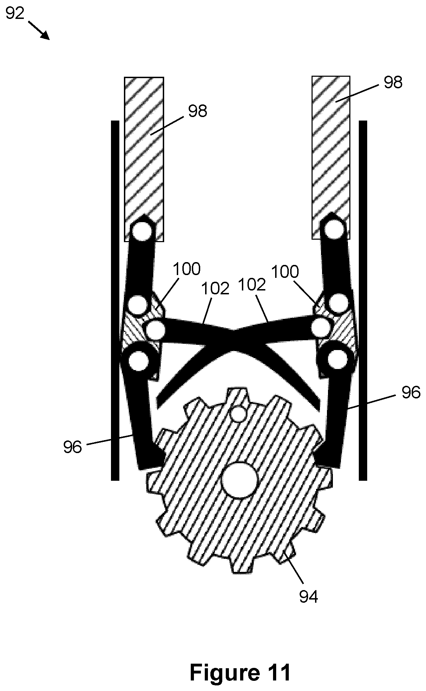

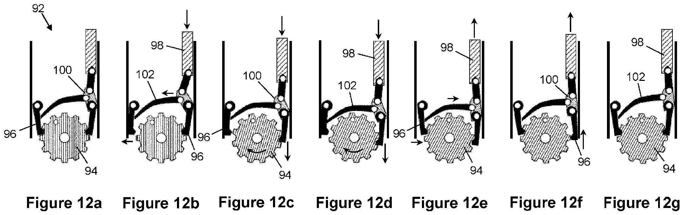

Turning next to Figure 11 and Figures 12a to 12g, these drawings show an orienting or drive mechanism 92 for turning the head 14, and hence the camera module 16 and the light emitter 18, relative to the probe 12 of the endoscope 10. The drive mechanism 92 is located near the distal end of the probe 12 and employs sliding fingers or pawls that could, for example, be powered using MEMS actuators.

Specifically, the drive mechanism 92 has a ratchet and pawl arrangement that comprises a ratchet wheel 94 coupled to the head 14, for example coaxially for direct drive or via the drive cable 26 of preceding embodiments. The drive mechanism 92 further comprises a pair of opposed pawls 96 that are movable as part of a chain or sequence of movements to engage with and apply torque to the ratchet wheel 94, thereby to drive and control angular stepwise movement of the ratchet wheel 94.

For this purpose, each pawl 96 is movable by a respective actuating rod 98 and linkage 100 to engage and disengage the ratchet wheel 94 and to apply torque to the ratchet wheel 94 when so engaged. A connecting arm 102 extending from each linkage 100 is driven by movement of that linkage 100 to act on the linkage 100 of the opposed pawl 96. In this way, extending one pawl 96 to advance the ratchet wheel 94 in a particular angular direction releases the other pawl 96 from the ratchet wheel 94 to free the rachet wheel 94 for that movement.

Figure 11 shows the complete drive mechanism 92 required to turn the ratchet wheel 94 in opposite angular directions. For ease of understanding, however, Figures 12a to 12g omit one of the actuating rods 98, linkages 100 and arms 102 to illustrate unidirectional angular movement of the ratchet wheel 94. It will nevertheless be clear how these features omitted from Figures 12a to 12g but shown in Figure 11 can be used to turn the ratchet wheel 94 in the opposite direction.

The sequence of operation shown in Figures 12a to 12g is as follows. Firstly, in the rest position shown in Figure 12a and corresponding to Figure 11, both pawls 96 are engaged with the ratchet wheel 94 to prevent angular movement of the head 14 relative to the probe 12.

Figure 12b shows the actuating rod 98 of one of the pawls 96 driven longitudinally toward the ratchet wheel 94 along a tangential axis that is offset laterally from the centre of the ratchet wheel 94. Initially, this movement pivots the linkage 100 relative to the actuating rod 98 and the pawl 96. That pivotal movement of the linkage 100 moves the arm 102 attached to that linkage 100 toward and against the opposed pawl 96. This movement of the arm 102 disengages the opposed pawl 96 from the ratchet wheel 94 to free the ratchet wheel 94 for movement.

Clockwise movement of the ratchet wheel 94 is then driven by further longitudinal movement of the pawl 96 that is coupled to the actuating rod 98 via the linkage 100, as shown in Figures 12c and 12d.

Next, the actuating rod 98 reaches the end of its longitudinal stroke and starts to return in the opposite longitudinal direction as shown in Figure 12e. Initially, this return movement pivots the linkage 100 in the opposite direction, pulling the arm 102 away from the opposed pawl 96. This allows the opposed pawl 96 to reengage the ratchet wheel 94, hence preventing angular movement of the ratchet wheel 94. Figure 12f then shows the actuating rod 98 retracting further, hence disengaging the pawl 96 coupled to the linkage 100 from the ratchet wheel 94. Further retraction of the actuating rod 98 pulls that pawl 96 anticlockwise, sliding across at least one tooth of the ratchet wheel 94 to reengage the ratchet wheel 94 at a relatively anticlockwise position as shown in Figure 12g.

The actuating rod 98 is now ready to repeat its longitudinal stroke if the ratchet wheel 94 is to be turned further in the clockwise direction. Alternatively, the actuating rod 98 can remain stationary if the ratchet wheel 94 is to be held at a fixed angular position or is to be driven in an anticlockwise direction by corresponding operation of the actuating rod 98, linkage 100 and arm 102 associated with the opposed pawl 96 as shown in Figure 11.

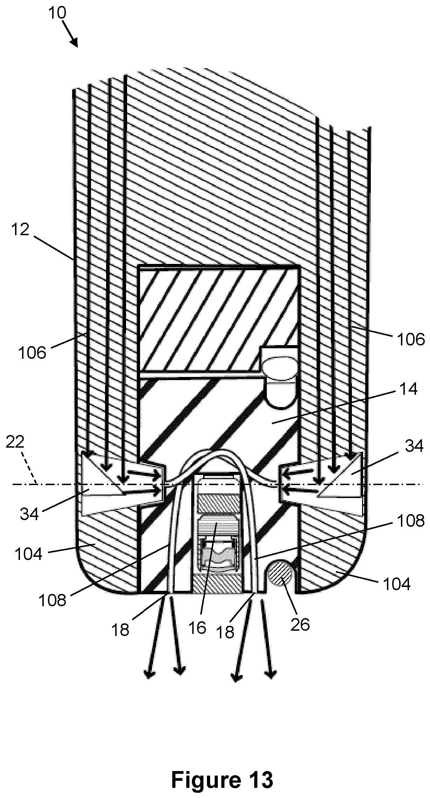

Figure 13 shows another example in which like numerals are again used for like features. In this example, the head 14 is not cantilevered from the probe 12 but is instead supported to turn about an axis of rotation 22 by, and between, a pair of laterally-spaced parallel arms 104 that extend distally from the probe 12. Again, the head 14 contains and encapsulates a camera module 16 and supports at least one light emitter 18 that is positioned to illuminate the field of view of the camera module 16. In this example, there are at least two light emitters 18, one each side of the camera module 16.

Optionally, as shown in this example, light is introduced into the head 14 from parallel longitudinal light paths 106 extending along the probe 12. The light is redirected from those light paths 106 toward the head 14 by mirrors, light pipes or prisms 34 in the respective arms 104, aligned with the axis of rotation 22 in mutual opposition about the head 14. Thus, light enters the head 14 from opposite sides along the axis of rotation 22. From there, light guides 108 such as optical fibres within the head 104 convey the light to respective light emitters 18.

In this example, the camera module 16 could be electrically powered by conventional means such as sliding contacts or electromagnetic induction. However, it will be apparent that the camera module 16 shown in Figure 13 could instead be powered by a photovoltaic arrangement using a portion of the light entering the head 14, for example using one or more light guides interposed between the camera module 16 and at least one of the arms 104 like the embodiment of Figures 1 to 3.

Again, features may be provided to facilitate robotic disassembly of the head 14 in a circular economy system, such as lines of weakness at which the head 14 can be cut or broken apart to access recyclable or reusable components encapsulated within.

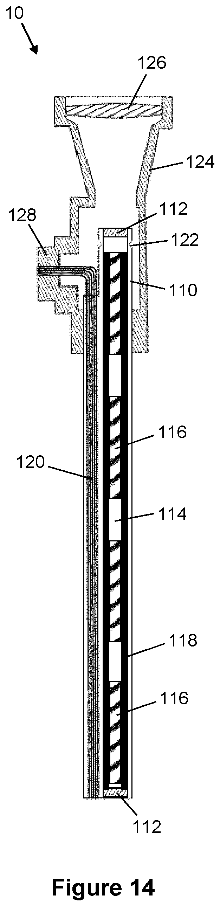

Moving on to Figures 14, 15 and 16, these drawings show examples useful for understanding the present disclosure in the form of endoscopes that resemble a traditional Hopkins^{®} scope.

In the endoscope 10 of Figure 14, a tubular capsule 110 has its ends closed with lenses or transparent end caps 112 to encapsulate a rod lens assembly 114. The rod lens assembly 114 comprises a longitudinal array of rod lenses 116 within a tubular housing 118, which may for example be of stainless steel. The metallic housing 118 of the rod lens assembly 114 lends stiffness to the longitudinal shaft of the endoscope 108, noting that the capsule 110 is a single-use component that may be moulded of polymer.

A lateral extension of the capsule 110 also houses and supports, but does not encapsulate, optical fibres 120 that extend parallel to the rod lens assembly 114. The optical fibres 120 terminate at the distal end of the endoscope 10 in a light emitter that is adjacent to the distal end cap 112 of the capsule 110. The distal end cap 112 serves as a distal lens of the endoscope 10.

The capsule 110 is encircled near its proximal end by a groove 122 defining a line of weakness for controlled disassembly, whereby the proximal end cap 112 can be removed or hinged away from the capsule 110. This allows the rod lens assembly 114 to be removed from the capsule 110 and reused. For example, the distal end cap 112 could be pressed proximally into the capsule 110 to push the rod lens assembly 114 proximally out of the capsule 110. That telescopic action could also break off or otherwise open the proximal end cap 112 if the proximal end cap 112 has not already been removed or opened. Preferably, the end caps 112 are spaced longitudinally from the rod lens assembly 114 to ensure that the rod lens assembly 114 is not damaged during removal from the capsule 110.

The capsule 110 is supported by a structural support 124 that surrounds and shrouds the proximal end of the capsule 110. The groove 122 is located within the support 124, which therefore protects the proximal end of the capsule 110 that is weakened by the groove 122. The capsule 110 must therefore be removed from the support 124 when access to the rod lens assembly 114 is required. The capsule 110 is then held so as to apply even force across the rod lens assembly 114 before a force is applied on the proximal side of the groove 122 to tear the capsule 110.

The support 124 also holds a proximal lens 126 in alignment with the rod lens assembly 114 and defines a connector 128 for coupling a light source to the optical fibres 120. The support 124 is preferably made from a biomaterial. Optionally, the optical fibres and the proximal lens could also be encapsulated in a similar manner to the rod lens assembly 114.

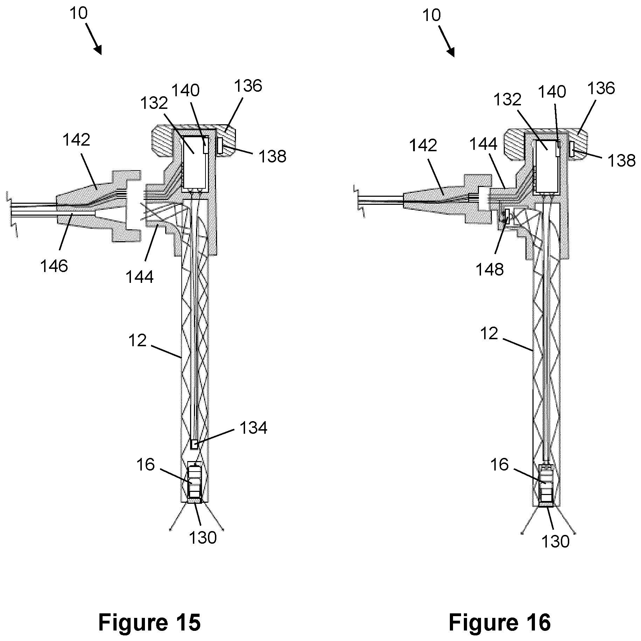

The endoscopes 10 shown in Figures 15 and 16 also resemble a traditional Hopkins^{®} scope, with optical fibre light guide illumination, but in this case they each have an integrated, encapsulated camera module 16 at a distal end to provide a chip-on-tip solution.

Preferably, as shown, the camera module 16 includes a variable-focus lens that is controllable from the proximal end of the endoscope 10. For example, an objective lens 130 in front of the image sensor of the camera module 16 would allow focusing of the camera module 16 by varying the distance between them.

A separate encapsulated control module 132 near the proximal end of each endoscope 10 controls focus of the camera module 16 and receives image data from the camera module 16. In Figure 15, the control module 132 receives data from an antenna 134 positioned close to the camera module 16 to receive that data wirelessly from the camera module 16. Conversely, the control module 132 has a wired data connection to the camera module 16 in Figure 16.

Focus of the camera module 16 may conveniently be controlled by a proximal knob 136 that acts on the control module 132. In these examples, the knob 136 interacts with the control module 132 without physical contact between them. For this purpose, the knob 136 has reference markers 138 whereby a sensor 140 of the control module 132 can monitor the movement of the knob 136.

In the endoscopes 10 of Figures 15 and 16, a tubular shaft or probe 12 serves as a structural element and as a light guide. Light is conveyed along the probe 12 by internal reflection, as shown, to emerge from the distal tip of the probe 12 around the camera module 16. In the embodiment of Figure 15, some of that light may power the camera module 16 via a photovoltaic cell. In the embodiment of Figure 16, the camera module 16 may be powered directly by a wired connection to the control module 132.

Power and data connections to the control module are made via cables 142 that couple to connectors 144 of the endoscopes 10 in Figures 15 and 16. In Figure 15, the cable 142 also includes fibre optics 146 to feed light from a remote light source, not shown, into the light guide of the probe 12 via the connector 144. Conversely, in Figure 16, the cable 142 provides electrical power to an onboard light source within the endoscope 10, exemplified here by an LED 148 that feeds light into light guide of the probe 12.

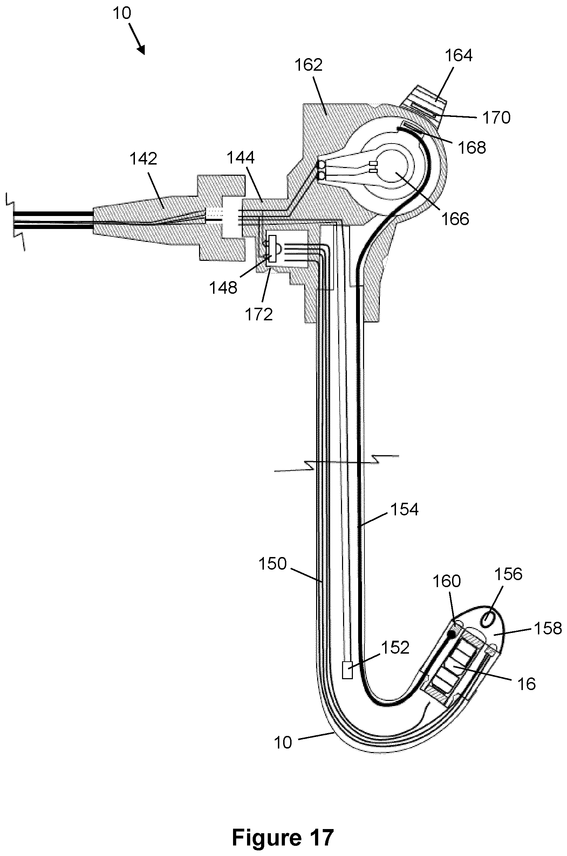

Turning next to Figure 17, the endoscope 10 of this example useful for understanding the present disclosure has a flexible tubular probe 12 that encloses optical fibres 150, a wireless communication module 152, and a steering wire 154 that is configured to bend the flexible probe 12 along its length. The probe 12 also accommodates a working channel that terminates in an exit opening 156 in a head component 158 at the distal tip of the probe 12.

In addition to defining the exit opening 158 for the working channel of the probe 12, the head component 158 encapsulates a camera module 16, directs illumination from the optical fibres 150 and provides a protective anchor for a plug 160 at the distal end of the steering wire 154. Some of the optical fibres 150 are directed to illuminate a photovoltaic cell that provides electrical power to the camera module 16. In conjunction with the probe 12 and a handle structure 162, the head component 158 also maintains protection of internal components of the endoscope 10 against ingress of contaminants.

The handle 162 includes a control element 164 to manoeuvre the steering wire 154 without direct physical interaction between the control element 164 and an internal mechanism 166 that is encapsulated within the handle 162. For this purpose, a sensor 168 of the mechanism 166 monitors movement of a reference marker 170 on the control element 164, causing the mechanism 166 to respond to such movement by pushing or pulling the steering wire 154 to the desired extent. Optionally, the mechanism 166 is assisted or powered by a motor. Points of weakness 172 may be provided for controlled disassembly of the handle 162 to extract the encapsulated mechanism 166 for reuse.

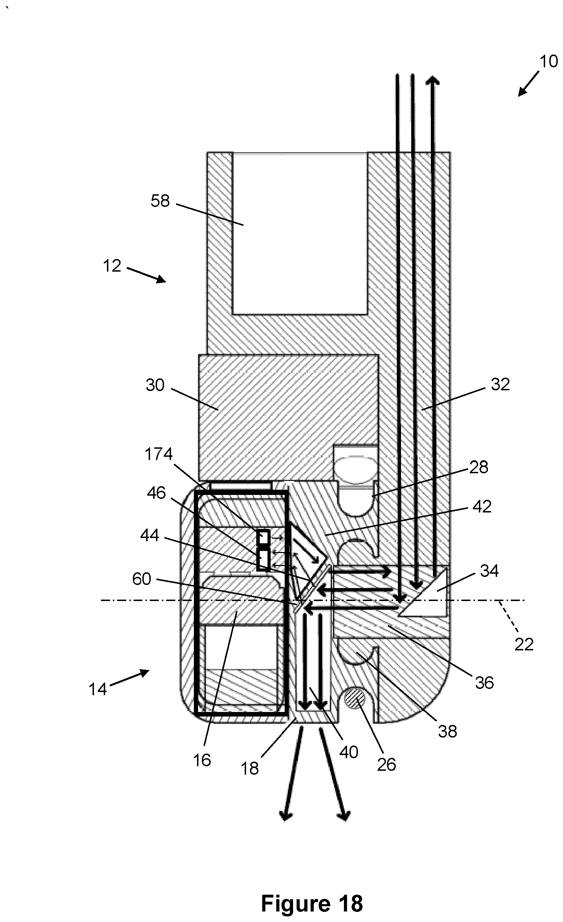

Moving on now to Figures 18 to 26, these drawings show various other wireless arrangements for powering the components of the head 14 and for receiving data from the head 14.

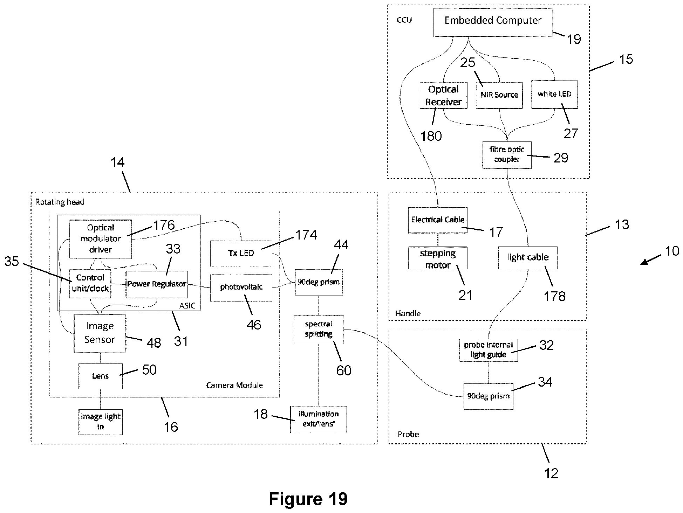

In the variant shown in longitudinal section in Figure 18 and as a block diagram in Figure 19, power and data are transferred to and from the head 14 optically. For this purpose, a data-transmitting LED 174 is shown in Figure 18 emitting data signals as light to the prism 44 in the head 14, which diverts that light to the prism 34 in the probe 12 and from there into the light guide 32.

The layout and components shown in the block diagram of Figure 19 are similar to those of the block diagram of Figure 5, and like numerals are used for like features. For brevity, the following description will focus on the main differences relative to Figure 5.

In the camera module 16 of Figure 19, the RF modulator 37 and the transmitting antenna 39 of Figure 5 are replaced by an optical modulator driver 176 in the ASIC 31 that feeds data signals to the transmitting LED 174.

In this example, the near-infrared source 25, the white LED 27 and the fibre optic coupler 29 are implemented in the camera control unit 15. Here, therefore, the coupler 29 is coupled to the light guide 32 in the probe 12 via a light cable 178 extending through the handle 13 to the probe 12. The light cable 178 not only conveys light toward the head 14 but also receives light from the LED 174 of the head 14 via the light guide 32, for example via the coupler 29 as shown. The light cable 41 conveys the received light to an optical receiver 180 in the camera control unit 15. The wavelength used for optical data transmission from the LED 174 is distinct from the wavelengths of white light transmission so that it can be distinguished and detected by the optical receiver 180. The optical receiver 180 feeds the resulting signals to the embedded computer 19 in the camera control unit 15.

Whilst the optical receiver 180, the near-infrared source 25, the white LED 27 and the fibre optic coupler 29 are implemented in the camera control unit 15 in this example, any or all of them could instead be implemented elsewhere outside the head 14, such as in the handle 13 or in the probe 14.

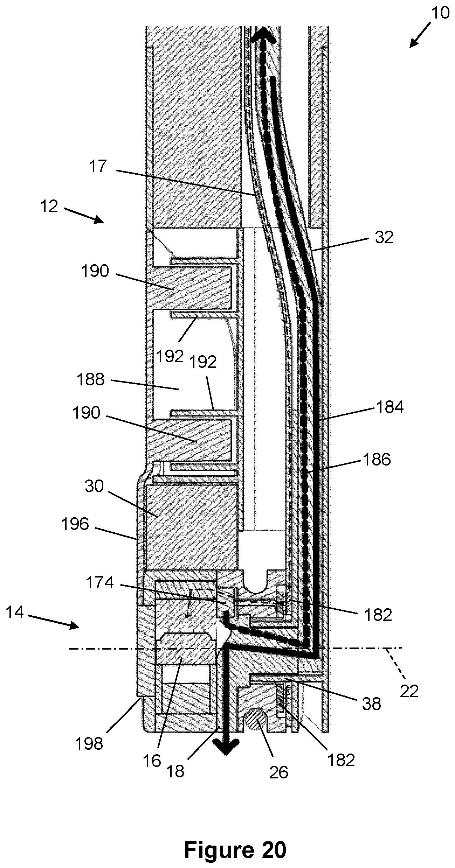

In the variants shown in Figures 20 to 26, data is transferred optically from the head 14 as in the preceding embodiment whereas power is transferred wirelessly to the head 14 by non-optical means such as by RF or by near-field inductive coupling. The latter possibility is illustrated in Figure 20, where power is transmitted inductively between mutually-confronting coils 182 that encircle the spigot 38 of the probe 12 relative to which the head 14 turns. One coil 182 is on the probe 12 and the opposed coil 182 is on the head 14.

The coil 182 of the probe 12 is connected to an electrical power cable 17 extending along the probe 12 in parallel relation to the light guide 32. The light guide 32 conveys illuminating light to the light emitter 18 of the head 14 on an outbound path 184. The light guide 32 also conveys light carrying a data signal from the data-transmitting LED 174 of the head 14 on an inbound path 186.

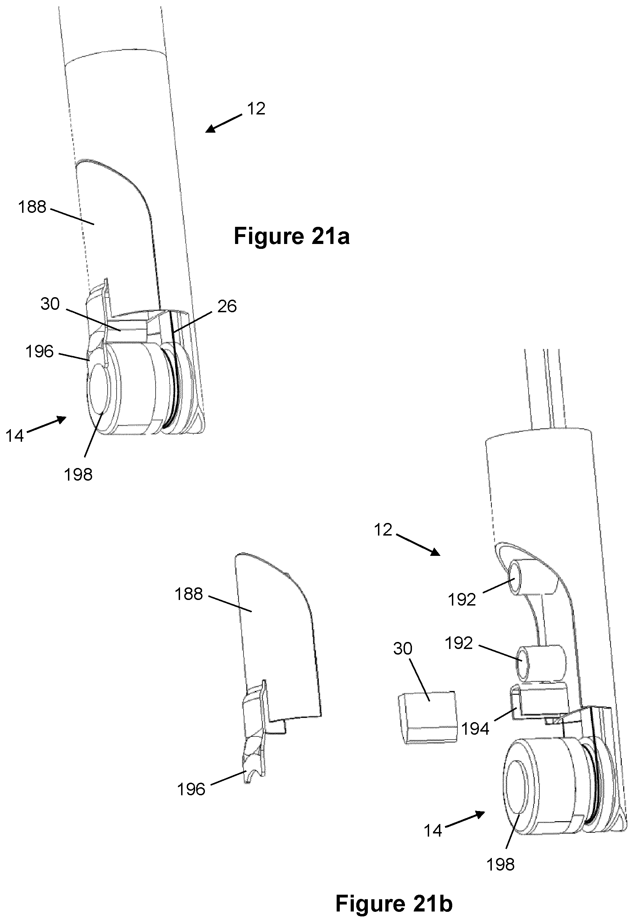

Figures 20, 21a and 21b also show a removable cover insert 188 that is held on the probe 12 by engagement of spigots 190 on the insert 188 within complementary sockets 192 on the probe 12. The insert 188 retains a cleaning module such as the wiper blade 30 in a receptable 194 that is integral with the probe 12. The insert 188 also has a concave hub bearing formation 196 that engages a complementary convex shoulder formation 198 centred on the axis of rotation 22 on an outer side of the head 14, opposed to the spigot 38. Thus, the insert 188 provides for assembly of the wiper blade 30 onto the probe 12 and for removal of the head 14 from the probe 12. The insert 188 also supports the wiper blade 30 and the head 12 in the assembled probe 12. Conversely, the insert 188 facilitates disassembly for circular manufacturing.

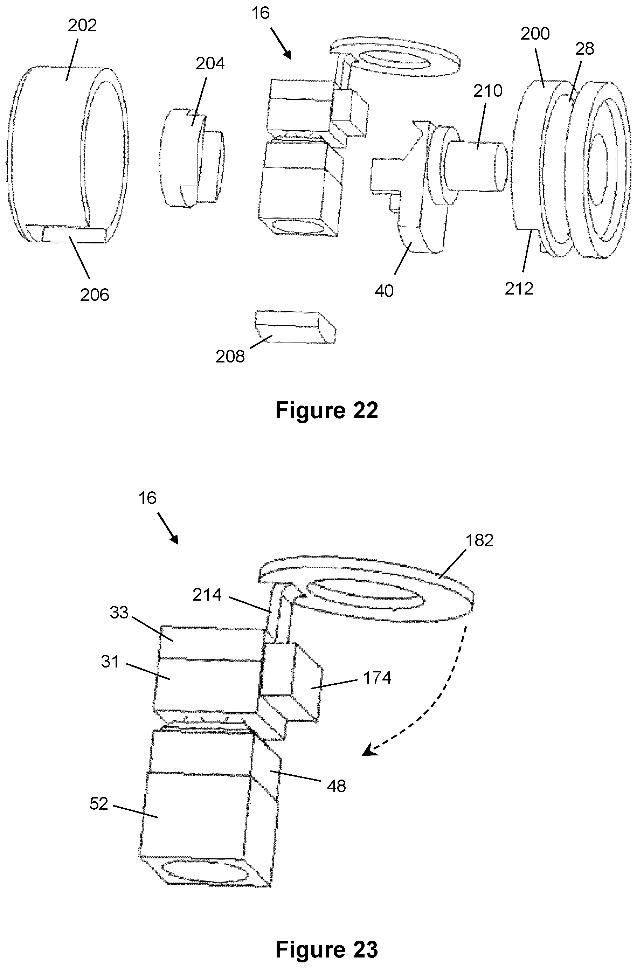

Figure 22 shows an exemplary assembly of components of the head 14. In this example, a pulley housing 200 and a camera housing 202 enclose and sandwich the camera module 16 and the light guide 40 in addition to an axis structure 204 that cooperates with the light guide 40 to locate the camera module 16 in the camera housing 202. The assembly further comprises a slot 206 in the camera housing 202 that receives a window 208 for admitting light to the camera module 16.

In a preferred sequence, the camera module 16 is assembled first with the window 208, the light guide 40 and the axis structure 204, which are preferably each designed to grip or locate the camera module 16 between them. The pulley housing 200 is then added to the assembly, followed by the camera housing 202. The pulley housing 200 is placed around a cylindrical projection 210 of the light guide 40 and the light guide 40 engages in a slot 212 in the pulley housing 200. This engagement locks the pulley housing 200 against angular movement relative to the remainder of the assembly.

The pulley housing 200 and the camera housing 202 could instead be formed over the other components of the assembly by an overmoulding process or by low-pressure injection moulding of adhesive, as will be described with reference to Figures 29(a) to 29(f).

The camera module 16 is illustrated in more detail in Figure 23, showing the lens 52, the image sensor 48, the ASIC 31 and its power regulator 33, the data-transmitting LED 174 and a power flex cable 214 comprising the coil 182 of the head 14. The power flex cable 214 is shown in Figures 22 and 23 in an initial position in which it is bent up to place the coil 182 at 90° to its final position after assembly. In that final position, the power flex cable 214 is bent down, as shown by the dashed arrow in Figure 23, such that the coil 182 lies over the cylindrical projection 210 of the light guide 40, parallel to the plane of the circumferential groove 28 in the pulley housing 200.

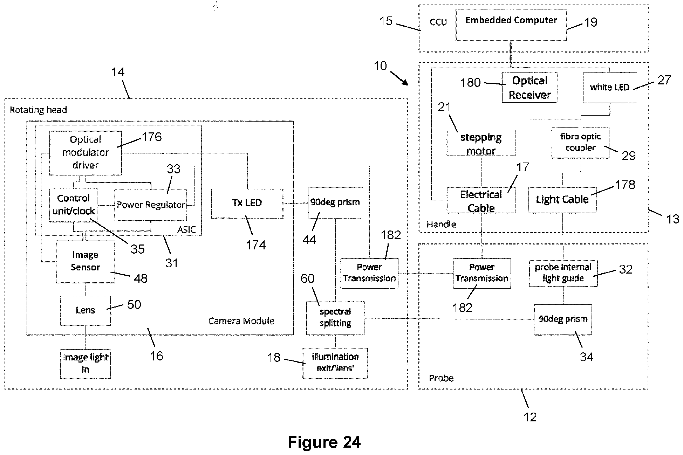

The layout and components shown in the block diagram of Figure 24 are similar to those of the block diagram of Figure 19, and like numerals are used for like features. The main difference relative to Figure 19 is the provision of the coils 182 that effect power transmission from the probe 12 to the head 14. The coil 182 of the probe 12 is shown here connected to the power cable 17 that also powers the stepping motor 21. Conversely, the coil 182 of the head 14 is shown connected to the power regulator 33 of the ASIC 31.

As the only light provided to the head 14 is for the purpose of illumination and as power is now supplied to the head 14 by other means, the near-infrared source 25 of Figure 19 is omitted. However, the light emitted from the data-transmitting LED 174 is still subject to spectral splitting or filtering at 60. This is to reduce reflection of light from the light emitter 18 back along the light guide 32, which could otherwise interfere with the ability of the optical receiver 180 to differentiate between the reflected light and the data signal from the LED 174.

Another difference of the block diagram of Figure 24 relative to Figure 19 is that the optical receiver 180, the LED 27 and the fibre optic coupler 29 are returned to the handle 13 from the camera control unit 15.

Further to help the optical receiver 180 to differentiate the data signal from light that could be reflected back along the light guide 32 from the probe 12 or the head 14, illumination of the LED 27 that emits white light may be synchronised with the frame rate of the image sensor 48. In particular, illumination of the LED 27 may be on only when the image sensor 48 takes a frame image and hence off in the intervals between frames. The interval periods in which illumination of the LED 27 is off can be used to transmit optical data without an overlap in signal, thus making signal processing easier. A similar provision can be made not only in the arrangement shown in Figures 20 to 24 but in any arrangement in which data is transmitted optically, including that of Figures 18 and 19.

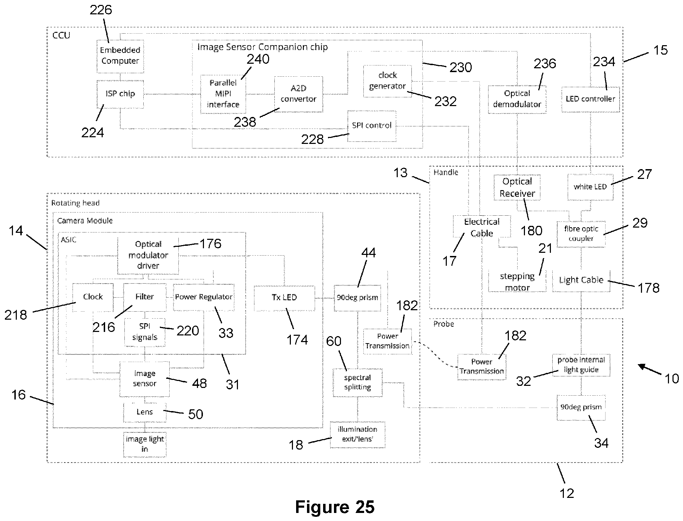

More generally, techniques used in modern electronics dictate that one-way data transmission from the image sensor 48 to the camera control unit 15 is not always an option and an element of two-way data communication may therefore be required. In this respect, Figure 25 develops the arrangement of Figure 24 to address two-way data transmission. Here, data is transmitted back to the camera control unit 15 using the light guide 32 as before but the wireless power transmission circuitry has a dual purpose of not only transmitting power but also transmitting data to the image sensor 48. That data 48 is in the form of a clock signal and a serial peripheral interface (SPI) control signal, namely an SPI write signal. The ASIC 31 of the camera module 16 comprises a filter 216 that differentiates between the clock signal at 218 and the SPI control signal at 220.

As the power transmission circuitry only provides for one channel, signal multiplexing is used. Specifically, to transmit a clock signal and SPI write data, the channel must transmit at least three different signal amplitude levels such as 0%, 50% and 100%. The clock signal is represented by 0% amplitude for the low portion of that signal and 50% amplitude for the high portion of that signal. For the short period of an SPI write data transfer, the 100% amplitude level is used to encode a 'high clock with high SPI write data signal', whereas the 50% amplitude level encodes a 'high clock with low SPI write data signal'. This is equivalent to amplitude modulation of the clock signal by the SPI write data signal.

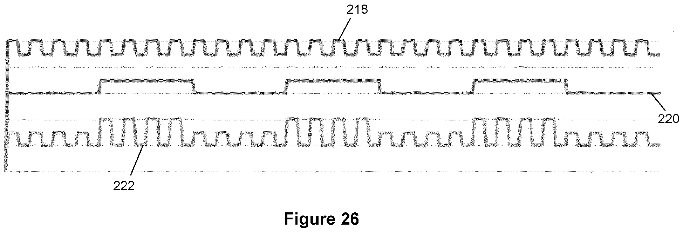

The multiplexing principle is illustrated in Figure 26, where a clock signal 218 and an SPI control signal 220 are combined into a multiplexed signal 222. As a bit of the SPI write data signal lasts for four clock cycles, modulation is simple.

Returning to Figure 25, this drawing further elaborates on the functionality of the camera control unit 15 in this context. An image signal processing chip 224 responsive to an embedded computer 226 drives an SPI controller 228 implemented on an image sensor companion chip 230. The SPI controller 228 feeds SPI write data to the electrical cable 17 that powers the coil 182 of the probe 14. The image sensor companion chip 230 also implements a clock 232 whose signal is also fed to the cable 17, multiplexed with the SPI write data generated by the SPI controller 228.

In a further variation, the LED 174 can act in two directions, both transmitting and receiving optical data. This allows the SPI control signal 220 to be transmitted optically to the rotating head.

The computer 226 also drives an LED controller 234 that, in turn, controls the LED 27 to generate illuminating white light. Reciprocally, an optical demodulator 236 receives and demodulates signals from the optical receiver 180 and feeds its output to the image signal processing chip 224 via an analog-to-digital converter 238 and a parallel mobile industry processor interface 240.

Turning next to Figures 27a and 27b, these exemplify an endoscope system 11 of the invention comprising three main components, namely a probe assembly 12 including a rotating head 14 at a distal end of a shaft 242; a handle assembly 13; and a camera control unit 15 connected to the handle assembly 13 by a cable 17. In this example, the handle assembly 13 and the camera control unit 15 are reusable whereas the probe assembly 12 is single-use or subject to circular manufacturing so as to be provided in a sterile state for each surgical procedure.

The probe assembly 12 has a cylindrical body 244 that supports and is surrounded by an over-sheath 246. The over-sheath 246 is secured to the body 244 by a series of O-rings 248 that encircle both the over-sheath 246 and the body 244. The over-sheath 246 can be pulled over the handle assembly 13 as shown in Figure 27b to provide a sterile barrier around the handle assembly 13.

The handle assembly 13 includes cables and connectors necessary to interface with the probe assembly 12 and the camera control unit 15. Optionally, the handle assembly 13 includes controls 250 for adjusting the head 14 and for initiating cleaning or wiping of the camera module 16. The handle assembly 13 may also include the aforementioned stepping motor 21 acting on a mechanism to rotate the head 14, along with electrical contacts, an optical receiver 180, LEDs and PCBs.

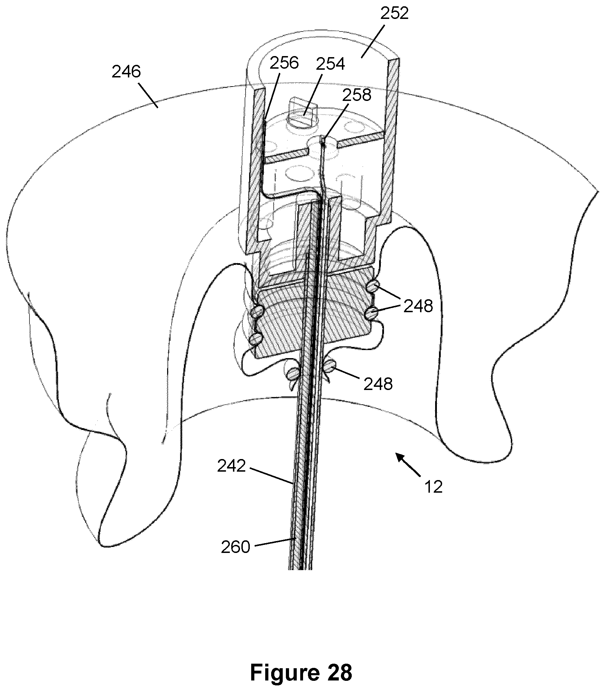

The probe assembly 12 includes conduits for conveying power, data, illumination and mechanical movement to and/or from the rotating head 14. With reference to the proximal end view of the probe assembly 12 of Figure 28, this shows features that interface with the handle assembly 13, namely a socket 252 for receiving a distal end of the handle assembly, a mechanical rotation interface 254 or dog clutch for receiving drive from the stepping motor 21, electrical contacts 256 and an optical interface 258. Figure 28 also shows that the shaft 242 of the probe 12 is of multi-lumen construction and is supported by an internal structural wire 260.

Turning finally to Figures 29a to 29f, these drawings illustrate ways of meeting the challenges of manufacturing an endoscope of the invention. In this respect, the extreme miniaturisation of the head 14, in particular, means that novel approaches are beneficial during manufacturing and assembly.

Figures 29a to 29f show a sequence of steps that deploy overmoulding techniques in a clean room assembly line for building a subassembly of an endoscope 10. Overmoulding may be done using micro injection moulding or low-pressure adhesive injection moulding techniques.

Figure 29a shows a distal structure of the probe 12 with a power flex cable 262 in place, a coil 182 of the power flex cable 262 encircling a spigot 38 that defines the axis of rotation 22. This assembly is positioned in a mould for overmoulding. Next, Figure 29b shows a power flex cable 214 of the camera module 16 and the light guide component 40 as they too would be assembled into the mould.

Figure 29c shows the pulley housing 200 now overmoulded on top of the abovementioned previously-placed components. Next, with reference to Figure 29d, the resulting assembly is placed in a second mould for overmoulding again, before which the camera module 16, axis structure 204 and window 208 components are positioned. This step also allows for an electrical connection to be made between the camera module 16 and the power flex cable 214 of the camera module 16.

Figure 29e shows the assembly ready for the second overmoulding process and Figure 29f shows the assembly with the resultant camera housing overmould 264 in place. This defines the head 14 assembled onto the distal structure of the probe 12, with the head 14 being rotatable on the spigot 38 of the distal structure. The tightness of the assembly prevents ingress of fluids past the spigot 38 and the pulley housing 200 interface.

Importantly, the camera module 16 that houses the most expensive components, such as electronic components comprising rare metals, is completely encapsulated in this process. This is advantageous as it protects the camera module 16 from contamination with prions or other contaminants that are difficult or impossible to remove, hence allowing for disassembly and reuse of the camera module 16 in a circular manufacturing process.

Many other variations are possible within the scope of the present invention which is defined by the appended claims.

For example, data communication and power transmission between parts of a surgical instrument could, in principle, be achieved by wireless techniques other than those described, such as magnetic resonance communication. However, electrical contacts on, or conductors between, the outer surfaces of adjoining capsules, modules or components could potentially be used for this purpose.

Data communication and power transmission in an instrument may be effected by daisy-chaining between successive capsules, modules or encapsulated components of the instrument. Thus, each capsule, module or component in the daisy-chain arrangement has a means of receiving data or power from an external source and/or a means of transmitting or conveying data or power to an external receiver. Such daisy-chain features are exemplified in this specification by the provisions for receiving light into the head to power the camera module and by the provisions for transmitting data from the camera module to an external receiver. In the case of RF communication, daisy-chaining is advantageous because the short transmission distance means that power can be kept low, making it easier to provide sufficient electrical energy and ensuring that RF radiation does not penetrate deep into the patient's body.

In some embodiments of the invention, external elements of the endoscope may be made from biodegradable materials, thus further reducing waste that would otherwise be sent for recycling or incineration. This is most suitable for structural parts such as handles. The biodegradable parts can form part of a capsule and be disassembled as previously described. Alternatively, a second internal layer can form a hermetic seal. In that case, the second internal layer could be of a non-structural nature and could be supported and protected by an outer biodegradable layer.

The scope of the present invention is defined by the appended claims.

## Claims

1. A medical scope (10) comprising:
an imaging head (14) supported by and rotatably movable relative to a body (12) about an axis of rotation (22) extending from the body, the imaging head including an image sensor (48) and a light emitter (18) for illuminating a field of view of the image sensor a light path extending from the body parallel to the axis of rotation to the light emitter, the light path including a light inlet of the imaging head opposed to a light outlet of the body across a wireless interface between the imaging head and the body; and
a light transmitter (174) in the imaging head, the light transmitter being coupled to the light path to transmit image data from the image sensor to the body across the interface.

2. The scope of Claim 1, wherein the light inlet and the light outlet are mutually opposed across a mount at which the imaging head is movably attached to the body.

3. The scope of Claim 1 or Claim 2, wherein the light inlet and the light outlet are disposed on said axis of rotation about which the imaging head can turn relative to the body.

4. The scope of Claim 3, wherein the light emitter is disposed between the interface and the image sensor in a direction parallel to the axis of rotation, at a position spaced radially from the axis of rotation.

5. The scope of any preceding claim, wherein the interface further comprises a wireless power transmission coupling effecting transmission of power from the body to the image sensor.

6. The scope of Claim 5, wherein the power transmission coupling comprises mutually-opposed coils, a first of those coils surrounding the light inlet of the imaging head and a second of those coils surrounding the light outlet of the body.

7. The scope of any of Claims 1 to 4, wherein the imaging head further comprises a photovoltaic generator for powering the image sensor from light transmitted across the interface.

8. The scope of any preceding claim, wherein the imaging head further comprises a filter in the light path, configured to block reflected light from the light emitter and to pass light transmitted from the light transmitter.

9. The scope of any preceding claim, further comprising a light source outside the imaging head and coupled to the light path to provide illuminating light to the light emitter, wherein the light source and the image sensor are synchronised such that the light source is on when the image sensor is capturing an image frame and the light source is off between image frames while the light transmitter transmits imaging data from the image sensor.

10. The scope of any preceding claim, further comprising an image sensor controller located outside the imaging head and being arranged to convey a control signal from the image sensor controller across the interface to the image sensor.

11. The scope of Claim 10, wherein the control signal comprises a clock signal multiplexed with a write signal.

12. The scope of Claim 10 or Claim 11, wherein the image sensor controller is connected to a wireless power transmission coupling of the interface to convey the control signal to the image sensor through that coupling.

13. The scope of any of Claims 10 to 12, wherein the image sensor controller drives an LED to convey the control signal to the image sensor as an optical control signal.

14. The scope of Claim 13, wherein the light transmitter in the imaging head is responsive to the optical control signal.

15. A method of operating a scope according to any of claims 1-14, the method comprising:
illuminating a field of view of the imaging head by conveying light along the light path parallel to said axis of rotation across the wireless interface from the body to the imaging head; and
transmitting image data optically along the light path across the interface from the imaging head to the body.

## Patentansprüche

1. Eine medizinische Skopievorrichtung (10), die Folgendes beinhaltet:
einen Bildgebungskopf (14), der durch einen Körper (12) gestützt wird und relativ zu diesem um eine Rotationsachse (22), die sich aus dem Körper erstreckt, rotierbar beweglich ist, wobei der Bildgebungskopf einen Bildsensor (48) und einen Lichtsender (18) zum Beleuchten eines Sehfelds des Bildsensors umfasst;
einen Lichtweg, der sich aus dem Körper parallel zu der Rotationsachse zu dem Lichtsender erstreckt, wobei der Lichtweg einen Lichteinlass des Bildgebungskopfs, der einem Lichtauslass des Körpers über eine drahtlose Schnittstelle zwischen dem Bildgebungskopf und dem Körper gegenüberliegt, umfasst; und
einen Lichtübermittler (174) in dem Bildgebungskopf, wobei der Lichtübermittler mit dem Lichtweg gekoppelt ist, um Bilddaten von dem Bildsensor über die Schnittstelle hinweg zu dem Körper zu übertragen.

2. Skopievorrichtung gemäß Anspruch 1, wobei der Lichteinlass und der Lichtauslass einander über eine Halterung hinweg, an der der Bildgebungskopf beweglich an dem Körper angebracht ist, gegenüberliegen.

3. Skopievorrichtung gemäß Anspruch 1 oder Anspruch 2, wobei der Lichteinlass und der Lichtauslass auf der Rotationsachse, um die sich der Bildgebungskopf relativ zu dem Körper drehen kann, angeordnet sind.

4. Skopievorrichtung gemäß Anspruch 3, wobei der Lichtsender zwischen der Schnittstelle und dem Bildsensor in einer parallel zu der Rotationsachse verlaufenden Richtung an einer radial von der Rotationsachse beabstandeten Position angeordnet ist.

5. Skopievorrichtung gemäß einem der vorhergehenden Ansprüche, wobei die Schnittstelle ferner eine drahtlose Stromübertragungskopplung, die die Übertragung von Strom von dem Körper zu dem Bildsensor bewirkt, beinhaltet.

6. Skopievorrichtung gemäß Anspruch 5, wobei die Stromübertragungskopplung einander gegenüberliegende Spulen beinhaltet, wobei eine erste dieser Spulen den Lichteinlass des Bildgebungskopfs umgibt und eine zweite dieser Spulen den Lichtauslass des Körpers umgibt.

7. Skopievorrichtung gemäß einem der Ansprüche 1 bis 4, wobei der Bildgebungskopf ferner einen Photovoltaikgenerator zum Versorgen des Bildsensors mit Strom aus Licht, das über die Schnittstelle hinweg übermittelt wird, beinhaltet.

8. Skopievorrichtung gemäß einem der vorhergehenden Ansprüche, wobei der Bildgebungskopf ferner ein Filter in dem Lichtweg beinhaltet, das dazu ausgelegt ist, von dem Lichtsender reflektiertes Licht zu blockieren und von dem Lichtübermittler übermitteltes Licht durchzulassen.

9. Skopievorrichtung gemäß einem der vorhergehenden Ansprüche, die ferner eine Lichtquelle beinhaltet, die außerhalb des Bildgebungskopfs liegt und mit dem Lichtweg gekoppelt ist, um dem Lichtsender Beleuchtungslicht bereitzustellen, wobei die Lichtquelle und der Bildsensor so synchronisiert sind, dass die Lichtquelle eingeschaltet ist, wenn der Bildsensor ein Einzelbild erfasst, und die Lichtquelle zwischen den Einzelbildern, während der Lichtübermittler Bilddaten von dem Bildsensor übermittelt, ausgeschaltet ist.

10. Skopievorrichtung gemäß einem der vorhergehenden Ansprüche, die ferner eine Bildsensorsteuerung beinhaltet, die sich außerhalb des Bildgebungskopfs befindet und dazu eingerichtet ist, ein Steuersignal von der Bildsensorsteuerung über die Schnittstelle hinweg zu dem Bildsensor zu transportieren.

11. Skopievorrichtung gemäß Anspruch 10, wobei das Steuersignal ein mit einem Schreibsignal gebündeltes Taktsignal beinhaltet.

12. Skopievorrichtung gemäß Anspruch 10 oder Anspruch 11, wobei die Bildsensorsteuerung mit einer drahtlosen Stromübertragungskopplung der Schnittstelle verbunden ist, um das Steuersignal durch diese Kopplung zu dem Bildsensor zu transportieren.

13. Skopievorrichtung gemäß einem der Ansprüche 10 bis 12, wobei die Bildsensorsteuerung eine LED antreibt, um das Steuersignal als ein optisches Steuersignal zu dem Bildsensor zu transportieren.

14. Skopievorrichtung gemäß Anspruch 13, wobei der Lichtübermittler in dem Bildgebungskopf auf das optische Steuersignal anspricht.

15. Ein Verfahren zum Bedienen einer Skopievorrichtung gemäß einem der Ansprüche 1-14, wobei das Verfahren Folgendes beinhaltet:
Beleuchten eines Sehfelds des Bildgebungskopfs durch Transportieren von Licht entlang des Lichtwegs parallel zu der Rotationsachse von dem Körper über die drahtlose Schnittstelle hinweg zu dem Bildgebungskopf; und
optisches Übermitteln von Bilddaten entlang des Lichtwegs von dem Bildgebungskopf über die Schnittstelle hinweg zu dem Körper.

## Revendications

1. Un endoscope médical (10), comprenant :
une tête d'imagerie (14) supportée par un corps (12) et mobile de manière rotative par rapport à celui-ci autour d'un axe de rotation (22) s'étendant depuis le corps, la tête d'imagerie comprenant un capteur d'image (48) et un émetteur de lumière (18) pour éclairer un champ de vision du capteur d'image
un trajet de lumière s'étendant du corps parallèle à l'axe de rotation vers l'émetteur de lumière, le trajet de lumière comprenant une entrée de lumière de la tête d'imagerie opposée à une sortie de lumière du corps à travers une interface sans fil entre la tête d'imagerie et le corps ; et
un transmetteur de lumière (174) dans la tête d'imagerie, le transmetteur de lumière étant couplé au trajet de lumière pour transmettre des données d'image du capteur d'image au corps à travers l'interface.

2. Endoscope selon la revendication 1, dans lequel l'entrée de lumière et la sortie de lumière sont mutuellement opposées à travers un support au niveau duquel la tête d'imagerie est fixée de manière mobile sur le corps.

3. Endoscope selon la revendication 1 ou la revendication 2, dans lequel l'entrée de lumière et la sortie de lumière sont disposées sur ledit axe de rotation autour duquel la tête d'imagerie peut tourner par rapport au corps.

4. Endoscope selon la revendication 3, dans lequel l'émetteur de lumière est disposé entre l'interface et le capteur d'image dans une direction parallèle à l'axe de rotation, à une position espacée radialement de l'axe de rotation.

5. Endoscope selon l'une quelconque des revendications précédentes, dans lequel l'interface comprend en outre un couplage de transmission de puissance sans fil assurant la transmission de puissance du corps au capteur d'image.

6. Endoscope selon la revendication 5, dans lequel le couplage de transmission de puissance comprend des bobines mutuellement opposées, une première de ces bobines entourant l'entrée de lumière de la tête d'imagerie et une seconde de ces bobines entourant la sortie de lumière du corps.

7. Endoscope selon l'une quelconque des revendications 1 à 4, dans lequel la tête d'imagerie comprend en outre un générateur photovoltaïque pour alimenter le capteur d'image à partir de la lumière transmise à travers l'interface.

8. Endoscope selon l'une quelconque des revendications précédentes, dans lequel la tête d'imagerie comprend en outre un filtre dans le trajet de lumière, configuré pour bloquer la lumière réfléchie de l'émetteur de lumière et pour passer la lumière transmise du transmetteur de lumière.

9. Endoscope selon l'une quelconque des revendications précédentes, comprenant en outre une source de lumière à l'extérieur de la tête d'imagerie et couplée au trajet de lumière pour fournir une lumière d'éclairage à l'émetteur de lumière, dans lequel la source de lumière et le capteur d'image sont synchronisés de telle sorte que la source de lumière est allumée lorsque le capteur d'image capture une trame d'image et que la source de lumière est éteinte entre les trames d'image, tandis que le transmetteur de lumière transmet des données d'imagerie du capteur d'image.

10. Endoscope selon l'une quelconque des revendications précédentes, comprenant en outre un contrôleur de capteur d'image situé à l'extérieur de la tête d'imagerie et agencé pour transmettre un signal de commande du contrôleur de capteur d'image à travers l'interface vers le capteur d'image.

11. Endoscope selon la revendication 10, dans lequel le signal de commande comprend un signal d'horloge multiplexé avec un signal d'écriture.

12. Endoscope selon la revendication 10 ou la revendication 11, dans lequel le contrôleur de capteur d'image est connecté à un couplage de transmission de puissance sans fil de l'interface pour transmettre le signal de commande au capteur d'image par ce couplage.

13. Endoscope selon l'une quelconque des revendications 10 à 12, dans lequel le contrôleur de capteur d'image active une DEL pour transmettre le signal de commande au capteur d'image en tant que signal de commande optique.

14. Endoscope selon la revendication 13, dans lequel le transmetteur de lumière dans la tête d'imagerie est sensible au signal de commande optique.

15. Procédé d'exploitation d'un endoscope selon l'une quelconque des revendications 1 à 14, le procédé consistant à :
illuminer un champ de vision de la tête d'imagerie en transmettant une lumière le long du trajet de lumière parallèle audit axe de rotation à travers l'interface sans fil du corps à la tête d'imagerie ; et
transmettre les données d'image optiquement le long du trajet de lumière à travers l'interface de la tête d'imagerie au corps.
